# EUROPEAN PATENT SPECIFICATION

(11) **EP 2 210 112 B1**
(45) Date of publication and mention of the grant of the patent: **08.02.2012**
(21) Application number: 08806601.4
(22) Date of filing: 15.10.2008
(51) Int. Cl.: G01N 33/68, G01N 33/569

(54) **DIAGNOSTIC METHODS FOR HIV INFECTION**
DIAGNOSTISCHE VERFAHREN FÜR HIV-INFEKTION
PROCÉDÉS DE DIAGNOSTIC DE L'INFECTION À VIH

(30) Priority: 15.10.2007 US 998953 P; 15.10.2007 US 998951 P
(43) Date of publication of application: 28.07.2010
(73) Proprietor: King's College London, London WC2R 2LS (GB); Baylor College Of Medicine, Houston, TX 77030 (US)
(72) Inventor: VYAKARNAM, Annapurna, London SE1 9RT (GB); ROWLEY, David, R., Houston, TX 77025 (US); RESSLER, Steven, Jon, Pearland, TX 77584 (US)
(74) Representative: Richards, William John
(86) International application number: PCT/GB2008/003477
(87) International publication number: WO 2009/050444

(56) References cited:
- HUNG HUYNH: "Suppression of ps20 expression in the rat uterus by tamoxifen and estrogens" ENDOCRINOLOGY, vol. 146, no. 5, May 2005 (2005-05), pages 2388-2396, XP002516203 ISSN: 0013-7227
- ALVAREZ R ET AL: "WFDC1/ps20 is a novel innate immunomodulatory signature protein of human immunodeficiency virus (HIV)-Permissive CD4(+) CD45RO(+) memory T cells that promotes infection by upregulating CD54 integrin expression and is elevated in HIV type 1 infection" JOURNAL OF VIROLOGY, vol. 82, no. 1, January 2008 (2008-01), pages 471-486, XP002516202 ISSN: 0022-538X

## Description

### Field Of The Invention

The invention relates to compositions and methods for the diagnosis of viral diseases such as HIV.

### Background Of The Invention

Viruses are ever-present pathogens capable of producing primary, latent, and recurrent infections which contribute to a variety of diseases. There is a critical need for rapid diagnostic methods for the efficient management of viral infections. Viruses may rely on host cell factors for infection, replication and/or pathogenesis and they may represent potential diagnostic targets. Of particular interest are host cell factors that mediate virus entry or facilitate replication and assembly.

Understanding the molecular biology of viral infection is the focus of considerable efforts in the prior art. However, despite these effort, many aspects of viral behaviour and the biology of infection and viral spread remain elusive. Moreover, although numerous molecular changes are known to take place following viral infection (ie. caused by viral infection), far less is known about the molecular determinants which are associated with and/or enable virus entry or the spread of viral infection. Thus, the prior art suffers from at least these shortcomings.

### Summary Of The Invention

The present inventors have discovered that the cell surface/secreted polypeptide ps20 is intimately associated with viral infection and viral spread. Moreover, the inventors have discovered that this protein can facilitate entry of free virus into mammalian cells as well as cell to cell spread of the virus. By comparing the behaviour of ps20 positive and ps20 negative cells when exposed to virus, and when in a cell-to-cell transfer model, the scientists have established that ps20 is a susceptibility factor. In other words, when ps20 is present, it is a marker of a cell that is more susceptible to viral infection than one which does not express ps20. Moreover, in studying these phenomena, the inventors have discovered a robust statistical association between the presence of ps20, either on cells or in blood plasma, with viral infection.

In other words, the inventors describe for the first time that ps20 may be assayed, and that a finding of elevated ps20 provides a reliable diagnostic marker of viral infection. In particular, these findings are of maximum importance for diagnosis of HIV infection, and also to determination of susceptibility to HIV infection. The present invention is based upon these remarkable findings.

Thus in one aspect the invention provides a method of aiding the diagnosis of a human immunodeficiency virus infection in a subject, said method comprising
(i) providing an in vitro sample sample from the subject
(ii) determining the level of ps20 in said sample
(iii) comparing the level of ps20 of (ii) with the level of ps2b in an uninfected reference sample,
wherein a higher level of ps20 in the sample from the subject compared to the uninfected reference sample indicates an increased likelihood of human immunodeficiency virus infection in said subject.

As well as human immunodeficiency virus, the invention may be applied to other viruses such as MHV1 (a SARS-like virus which can affect the lung), CVB3 (a cardiotropic virus which can affect the heart); thus the general applicability of the method of the invention is demonstrated. Suitably the virus is human immunodeficiency virus.

Suitably the sample comprises a sample of CD4+ T cells from the subject. Suitably determining the level of ps20 in said sample comprises determining the level of ps20 expression of said T cells.

ps20 expression may refer to gene expression e.g. expression assessed at the nucleic acid level. Alternatively, and more suitably, ps20 expression refers to expression of the ps20 polypeptide (whether internally or externally).

Also disclosed is a method of aiding the diagnosis of a human immunodeficiency virus infection in a subject, said method comprising
(i) providing a sample of CD4+ T cells from the subject
(ii) determining the level of ps20 expression in said T cells
(iii) comparing the level of ps20 expression of (ii): with the level of ps20 expression in an uninfected reference sample,
wherein a higher level of ps20 expression in the sample from the subject compared to the uninfected reference sample indicates an increased likelihood of human immunodeficiency virus infection in said subject.

In another aspect, the invention relates to a method as described above, wherein the sample comprises a sample of blood plasma from said subject. In this embodiment the ps20 analysed comprises secreted or extracellular ps20. Suitably the ps20 level is determined by ELISA assay of the blood plasma sample. Exemplary ELISA assays are set out in the examples section below.

Suitably the sample may comprise a sample of nucleic acid from said subject, and wherein determining the level of ps20 in said sample comprises determining the amount of ps20 transcript present in said sample.

Suitably the level of ps20 expression in said T cells is determined by assaying binding by an anti-ps20 antibody. Suitably this may be read out using flow cytometry. Exemplary systems for such readouts are presented in the examples section.

In another aspect, the invention relates to a method of assessing susceptibility of a subject to human immunodeficiency virus infection, said method comprising
(i) providing an in vitro sample sample from the subject
(ii) determining the level of ps20 in said sample
(iii) comparing the level of ps20 of (ii) with the level of ps20 in a normal reference sample,
wherein a higher level of ps20 in the sample from the subject compared to the normal reference sample indicates an increased susceptibility to human immunodeficiency virus infection in said subject.
Also disclosed is a method of assessing susceptibility of a subject to human immunodeficiency virus infection, said method comprising
(i) providing a sample of CD4+ T cells from the subject
(ii) determining the level of ps20 expression in said T cells
(iii) comparing the level of ps20. expression of (ii) with the level of ps20 expression in a normal reference sample,
wherein a higher level of ps20 expression in the sample from the subject compared to the reference sample indicates an increased susceptibility to human immunodeficiency virus infection in said subject.

Suitably when the means of detection of ps20 comprises an antibody, said antibody comprises monoclonal anti-ps20 antibody such as the IG7 monoclonal antibody.

Further suitable reagents for ps20 detection are set out below.

### Infection and Susceptibility

As is disclosed herein, ps20 exhibits a strong correlation with virus infection. In short, increased ps20 shows or infers increased virus/viral infection. Of course, in other aspects of the invention, a link between ps20 and susceptibility to viral infection is shown. For example, we describe experiments in which a ps20 positive and a ps20 negative cell mixture is exposed to virus. Following this exposure, it is clearly shown that the ps20 positive cells have an increased infection compared to the ps20 negative cells which have a lower infection. These results clearly show that ps20 presence is an indicator of susceptibility of the cell to viral infection.

Notwithstanding the findings and disclosures in connection with susceptibility, it should be noted that it is a key part of the present invention that ps20 is associated with viral infection. Moreover, the data presented in the examples section clearly demonstrate through several scientific perspectives that ps20 is a robust marker of viral infection. Indeed, it is even demonstrated that human patients harbouring the virus show increased ps20 levels. Thus, the marker has been validated as a marker of viral infection *in vivo* in human subjects.

It is important to note that cells may become infected with virus through entry of the free virus into the cell, or by spreading of the virus from cell to cell without necessarily passing through a free virus stage. These two modes by which an uninfected cell can become infected are both affected by the ps20 status of the cell. Not only are ps20 high cells more susceptible to infection by free virus, ps20 high cells are also more susceptible to cell-cell transfer of the virus. Again, this is experimentally demonstrated in the example section. In summary, if an infected cell is taken and exposed to ps20 high cells or to ps20 low cells, cell to cell transfer of the virus is more effective into ps20 high cells.

As is set out in more detail in the example section, *in vitro* experiments using a mixture of ps20 high and ps20 low cells exposed to virus consistently show that the virus passes more easily into ps20 high cells. These findings confirm the predictive value of assessing ps20 levels on cells, thereby experimentally validating the invention.

Numerous embodiments of the invention call for the measurement of ps20 levels. These ps20 levels are then used to make inferences about the likelihood of viral infection, or the infected status of the subject from which those samples were taken. ps20 is a cell surface (cell-associated) protein as well as being a secreted protein. It is demonstrated herein that the presence of elevated ps20 on the cell surface (ie, cell-associated ps20) correlates with infection. In other words, the finding of a higher level of ps20 on the cell surface makes the robust statistical inference of a greater likelihood of viral infection. The secreted ps20 is also a very useful marker of viral infection. Typically, secreted ps20 may be determined by measuring the ps20 levels in plasma, for example, by ELISA assay. In this setting, it is also clearly experimentally demonstrated that secreted ps20 (eg, plasma ps20) is a robust statistical marker of viral infection.

Of course, it may be desired to measure "total" ps20. For example, this may be done by measurement of ps20 RNA levels such as mRNA levels. This also represents a robust statistical marker of viral infection. Indeed, in some embodiments, it may be desirable or easer to obtain nucleic acid sample in order to perform total mRNA analysis. However, more typically, assay of ps20 polypeptide level is

It is important to note that numerous molecular markers are known to go up or be elevated following viral infection, or as a consequence of viral infection. However, the number of markers known to exert some control over viral infection as ps20 is shown to do, is extremely few. Thus, ps20 is identified as a very significant and important biological marker by virtue of its functional properties in relation to viral infection. These biological properties were not previously known or ascribed to ps20 in the prior art. Thus, the present invention discloses a novel and important biological function to ps20 in the context of viral infection.

### Samples

For assay of secreted ps20, blood plasma is a most suitable sample. An advantage of using blood plasma as a sample in accordance with the invention is that it is often regarded as routine to measure virus load in plasma during analysis of potentially infected blood samples. Therefore, in one aspect the invention relates to a combination involving the assay of ps20 levels in plasma together with the parallel measurement of virus load in plasma.

Peripheral T-cells represent an exemplary sample according to the present invention. These are susceptible of ps20 analysis by intracellular staining as well as cell surface staining. Such staining is easily analysed and quantified by flow cytometry. Furthermore, it is an advantage of using peripheral T-cells as the sample according to the present invention in that it is established in the art to make CD4 positive T-cell counts in order to understand more about progression of the disease. Thus, in one aspect, the invention relates to a combination of quantification of ps20 on or in peripheral T-cells, combined with a total CD4 positive cell count.

It is also possible to use extracted nucleic acid such as extracted RNA as a sample according to the present invention. This has the advantage of being easy to extract and to manipulate *in vitro.*

### Assays

Numerous assays are described in the example section of this application. One such assay is the assay of nucleic acid such as RNA, for example to quantify the amount of ps20 transcript present in the sample, suitably with normalisation in order to obtain a value for the approximate, number of transcripts per cell. This is particularly suitable when the sample comprises nucleic acid.

Intracellular staining of ps20 polypeptide may be used in order to assay ps20 levels according the present invention.

Extracellular staining of ps20 such as staining of cell surface ps20 may be used as a convenient way of quantifying ps20 levels according the present invention.

Clearly, any technique involving immunostaining of ps20 (whether intracellular or extracellular (cell surface)) may be conveniently combined with flow cytometry analysis in order to assist in data collection.

Most suitably, an ELISA-based assay is used to quantify ps20 according to the present invention. This has the advantage of being cheap to run, has the further advantage of being rapid to analyse, and is especially suitable when the sample comprises blood plasma.

### Reference Sample

The reference sample may be any suitable comparable sample to that being analysed. Suitably the sample is obtained from a normal individual. Suitably the sample ism obtained from an uninfected individual, ie, an individual who is known not to harbour the virus of interest.

In some embodiments, the reference sample may be comprised by a previously determined reference value, for example a particular molarity of ps20 or a particular mass of ps20 detected. However, more preferably the assays of the invention each comprise a reference sample, and ps20 levels are determined in a relative manner by comparison to the reference sample. This embodiment has the advantage of avoiding possible confounding of the results by determination of varying absolute values for the reference sample when the reference sample comprises a reference value. By always incorporating a reference sample into the assay being conducted, then a more robust and reliable indication of whether the amount of ps20 in the sample of interest is higher or lower than that found in the reference sample may be consistently obtained. For this reason, suitably the assays of the invention always comprise a reference sample determined in parallel to the sample of interest.

Suitably the invention relates to the diagnosis of human immunodeficiency virus. Suitably the virus of the invention is human immunodeficiency virus.

### Antibodies

Any antibody reactive with ps20 such as those raised against ps20 polypeptide or a fragment thereof may find application in the present invention. This includes polyclonal antibodies raised against the whole ps20 protein. This also includes monoclonal antibodies raised against whole ps20 polypeptide. This may also include monoclonal antibodies raised against particular epitopes or peptides taken from within the ps20 polypeptide, as is explained in more detail below.

Suitably, the antibody is one raised against one or more of the particular ps20 peptides disclosed herein. In particular, reference is made to the examples section and to the peptides disclosed therein such as peptides covering epitopes of particular interest are identified as 253 (amino acid {aa} 51-65 of the ps20 sequence) and peptide 254 (aa 206-220 of the ps20 sequence). In addition, two peptides that were found to bind strongly to the anti-ps20 monoclonal antibody were tested and these were identified as peptide 555 (aa 21-35) and peptide 556 (aa 91-105). It should be noted that peptide 555 (representing amino acids 21 to 35 of ps20) represents one of the most useful epitopes against which antibodies may be raised. Thus, suitably the antibody is an antibody which reacts with a peptide comprising amino acids 21 to 35 of human ps20. A most preferred example of this is the IG7 monoclonal antibody.

IG7 antibody is available via Baylor College of Medicine, USA.

Of course, it is possible to make synthetic antibodies. Ideally, when the antibody is a synthetic antibody, said antibody would have the variable region sequences of the IG7 monoclonal antibody. Of course, it is a straightforward matter to determine the amino acid sequence of that antibody, and then to manufacture the synthetic antibody using any of the numerous commercially available synthesising services.

Throughout the aspects and embodiments of this invention, suitably the virus or viral infection is human immunodeficiency virus or human immunodeficiency virus infection.

The invention relates to novel markers for viral diseases and compositions comprising same. The invention also relates to methods for assessing the status of CD4 T cells for susceptibility to viral diseases, and methods for the diagnosis of viral diseases. In particular, the present inventors have found that ps20 polypeptides and polynucleotides encoding the ps20 polypeptides, have application in the determination of the permissive or resistant status or phenotype of CD4 T cells for viral disease, as well as for diagnosis, monitoring (i.e. monitoring progression or therapeutic treatment), or classification of viral diseases, or as markers before or after treatment or after relapse.

Ps20 polypeptides including native-sequence polypeptides, isoforms, chimeric polypeptides, all homologs, fragments, and precursors of the polypeptides, as well as modified forms of the polypeptides and derivatives, are referred to herein as "ps20 polypeptide(s)". Polynucleotides encoding ps20 polypeptides are referred to herein as "pus20 polynucleotide(s)" or "polynucleotides encoding ps20 polypeptide(s)". The ps20 polypeptides and ps20 polynucleotides are sometimes collectively referred to herein as "ps20 marker(s)".

In accordance with methods of the invention, CD4 T cells can be assessed or characterized for susceptibility to a viral disease, for example by detecting in a sample of CD4 T cells the presence of (a) a ps20 polypeptide or fragment thereof; (b) a polypeptide which interacts with, or is modulated by a ps20 polypeptide; (c) a transcribed nucleic acid or fragment thereof having at least a portion with which a ps20 polynucleotide is substantially identical; and/or (c) a transcribed nucleic acid or fragment thereof, wherein the nucleic acid hybridizes with a ps20 polynucleotide.

In an aspect, a method is disclosed for characterizing CD4 T cells in a subject as permissive for a viral disease comprising:
(a) obtaining from the subject a sample comprising CD4 T cells;
(b) detecting or identifying in the sample ps20 polypeptides or ps20 polynucleotides; and
(c) comparing the detected amount with an amount detected for a standard.

The ps20 markers can be used to identify selected CD4 T cells that are characteristic of different stages of a viral disease.

In an aspect, a method is disclosed for detecting ps20 polypeptides or ps20 polynucleotides associated with viral disease in a patient comprising:
(a) obtaining from a patient a sample which is suspected of containing a ps20 polypeptide or ps20 polynucleotide;
(b) detecting in the sample ps20 polypeptides or ps20 polynucleotides; and
(c) comparing the detected amount with an amount detected for a standard.

The term "detect" or "detecting" includes assaying, imaging or otherwise establishing the presence or absence of the target ps20 polypeptides or polynucleotides, subunits thereof, or combinations of reagent bound targets, and the like, or assaying for, imagine, ascertaining, establishing, or otherwise determining one or more factual characteristics of CD4 T cells or viral diseases, including HIV and influenza, or similar conditions. The term encompasses diagnostic, prognostic, and monitoring applications for the ps20 polypeptides and ps20 polynucleotides.

Also disclosed is a method of assessing whether a patient is afflicted with or has a pre-disposition for a viral disease, in particular HIV or influenza, the method comprising comparing:
(a) levels of ps20 polypeptides or ps20 polynucleotides associated with the viral disease in a sample from the patient; and
(b) normal levels of ps20 polypeptides or ps20 polynucleotides in a sample of the same type obtained from control patients not afflicted with the disease, wherein altered levels of the ps20 polypeptides or the ps20 polynucleotides relative to the corresponding normal levels of ps20 polypeptides or ps20 polynucleotides is an indication that the patient is afflicted with the viral disease.

In another particular embodiment of a method of the invention for assessing whether a patient is afflicted with or has a pre-disposition for a viral disease, higher levels of ps20 polypeptides or ps20 polynucleotides in a sample relative to the corresponding normal levels is an indication that the patient is afflicted with viral disease.

Also disclosed is a method for diagnosing a viral disease in a subject comprising:
(a) obtaining from the subject a biological sample suspected of containing ps20 polypeptides or ps20 polynucleotides;
(b) assaying for ps20 polypeptides or ps20 polynucleotides in the sample; and
(c) comparing the amount of ps20 polypeptides or ps20 polynucleotides assayed to a predetermined standard, where detection of amounts of ps20 polypeptides or ps20 polynucleotides that differ significantly from the standard indicates a viral disease or susceptibility or predisposition to a viral disease. A significant difference between the amounts of ps20 polypeptides or ps20 polynucleotides in a subject and amounts of ps20 polypeptides or ps20 polynucleotides from samples of a normal subject is an indication that the subject is afflicted with or is susceptible to or has a predisposition to a viral disease. In an embodiment the amount of ps20 markers detected is greater than that of a standard and is indicative of a viral disease, in particular HIV or influenza.

A method for screening a subject for a viral disease is disclosed comprising (a) obtaining a biological sample from a subject; (b) detecting the level of ps20 polypeptides or ps20 polynucleotides associated with the disease in said sample; and (c) comparing said level of ps20 polypeptides or ps20 polynucleotides detected to a predetermined standard, where detection of a level of ps20 polypeptides or ps20 polynucleotides that differs significantly from the standard indicates a viral disease or susceptibility or predisposition to a viral disease. A significant difference between the levels of ps20 polypeptides or ps20 polynucleotides in a patient and the normal levels is an indication that the patient is afflicted with or is susceptible to or has a predisposition to a viral disease. In an example the level of ps20 polypeptide(s) detected is greater than that of a standard and is indicative of a viral disease, in particular HIV or influenza.

In another aspect, disclosed herein is a method for assessing the aggressiveness or indolence of a viral disease, in particular HIV or influenza, the method comprising comparing:
(a) levels of ps20 polypeptides or ps20 polynucleotides associated with the viral disease in a patient sample; and
(b) normal levels of the ps20 polypeptides or the ps20 polynucleotides in a control sample.

A significant difference between the levels in the sample and the normal levels is an indication that the viral disease is aggressive or indolent. In an example, the levels of ps20 polypeptides or ps20 polynucleotides are higher than normal levels.

In an aspect, disclosed is a method for monitoring the progression of a viral disease, in particular HIV or influenza, in a patient the method comprising:
(a) detecting ps20 polypeptides or ps20 polynucleotides associated with the disease in a sample from the patient at a first time point;
(b) repeating step (a) at a subsequent point in time; and
(c) comparing the levels detected in (a) and (b), and therefrom monitoring the progression of the viral disease.

The invention further relates to a method of assessing the efficacy of a therapy for inhibiting a viral disease in a patient. A method of the invention comprises comparing: (a) levels of ps20 polypeptides or ps20 polynucleotides in a sample from the patient obtained from the patient prior to providing at least a portion of the therapy to the patient; and (b) levels of ps20 polypeptides or ps20 polynucleotides in a second sample obtained from the patient following therapy.

A significant difference between the levels of ps20 polypeptides on ps20 polynucleotides in the second sample relative to the first sample is an indication that the therapy is efficacious for inhibiting a viral disease.

In an embodiment, the method is used to assess the efficacy of a therapy for inhibiting a viral disease, where lower levels of ps20 polypeptides or ps20 polynucleotides in the second sample relative to the first sample, is an indication that the therapy is efficacious for inhibiting the disease.

The "therapy" may be any therapy for treating a viral disease including but not limited to therapeutics, immunotherapy, and gene therapy. Therefore, the method can be used to evaluate a patient before, during, and after therapy.

Certain methods of the invention employ binding agents (e.g. antibodies) that specifically recognize ps20 polypeptides. In an aspect, the invention provides methods for determining the presence or absence or severity of a viral disease, in particular HIV or influenza, in a patient, comprising the steps of (a) contacting a biological sample obtained from a patient with one or more binding agent that specifically binds to one or more ps20 polypeptides; and (b) detecting in the sample an amount of ps20 polypeptides that bind to the binding agent, relative to a predetermined standard or cut-off value, and therefrom determining the presence or absence or severity of a viral disease in the patient.

Disclosed further is a method for diagnosing a viral disease in a subject by quantitating one or more ps20 polypeptides in a biological sample from the subject comprising (a) reacting the biological sample with one or more binding agent specific for the ps20 polypeptides (e.g. an antibody) that are directly or indirectly labelled with a detectable substance; and (b) detecting the detectable substance.

Disclosed is also a method for monitoring a viral disease in a subject by quantitating one or more ps20 polypeptides in a biological sample from the subject comprising (a) reacting the biological sample with one or more binding agent specific for the ps20 polypeptides (e.g. an antibody) that are directly or indirectly labelled with a detectable substance; and (b) detecting the detectable substance.

Also disclosed is a method for using an antibody to detect expression of one or more ps20 polypeptides in a sample, the method comprising: (a) combining antibodies specific for one or more ps20 polypeptides with a sample under conditions which allow the formation of antibody:marker complexes; and (b) detecting complex formation, wherein complex formation indicates expression of a ps20 polypeptide in the sample. Expression may be compared with standards and is diagnostic of a viral disease, in particular HIV or influenza.

In an embodiment quantitated levels of ps20 polypeptides are compared to levels quantitated for control subjects (e.g. normal) without a viral disease wherein an increase in ps20 polypeptide levels compared with the control subjects is indicative of the viral disease.

A particular embodiment of the invention comprises the following steps
(a) incubating a biological sample with first antibodies specific for one or more ps20 polypeptides which are directly or indirectly labeled with a detectable substance, and second antibodies specific for one or more ps20 polypeptides which are immobilized;
(b) detecting the detectable substance thereby quantitating ps20 polypeptides in the biological sample; and
(c) comparing the quantitated ps20 polypeptides with levels for a predetermined standard.

The standard may correspond to levels quantitated for samples from control subjects without a viral disease, with a different disease stage, or from other samples of the subject. In an embodiment, increased levels of ps20 polypeptides as compared to the standard may be indicative of a viral disease.

Other methods of the invention employ one or more polynucleotides capable of hybridizing to one or more ps20 polynucleotides. Thus, the present invention relates to a method for diagnosing or monitoring a viral disease in a sample from a subject comprising isolating nucleic acids, preferably mRNA, from the sample; and detecting ps20 polynucleotides in the sample. The presence of different levels of ps20 polynucleotides in the sample compared to a standard or control may be indicative of disease, disease stage, and/or prognosis.

In an embodiment of the invention, ps20 polynucleotide positive samples (e.g. higher levels of the ps20 polynucleotides compared to a control normal) are a negative diagnostic indicator. Positive samples can be indicative of a viral disease, advanced stage disease, and/or lower overall survival.

Disclosed are methods for determining the presence or absence or severity of a viral disease in a subject comprising detecting in the sample levels of nucleic acids that hybridize to one or more ps20 polynucleotides associated with the disease, comparing the levels with a predetermined standard or cut-off value, and therefrom determining the presence or absence or severity of the viral disease in the subject. In an example, the invention provides methods for determining the presence or absence or severity of a viral disease in a subject comprising (a) contacting a sample obtained from the subject with oligonucleotides that hybridize to one or more ps20 polynucleotides; and (b) detecting in the sample a level of nucleic acids that hybridize to the ps20 polynucleotides relative to a predetermined cut-off value, and therefrom determining the presence or absence or severity of the viral disease in the subject.

Within certain examples, the amount of polynucleotides that are mRNA are detected via polymerase chain reaction using, for example, oligonucleotide primers that hybridize to one or more ps20 polynucleotides, or complements of such ps20 polynucleotides. Within other embodiments, the amount of mRNA is detected using a hybridization technique, employing, oligonucleotide probes that hybridize to one or more ps20 polynucleotides, or complements thereof.

When using mRNA detection, the method may be carried out by combining isolated mRNA with reagents to convert to cDNA according to standard methods; treating the converted cDNA with amplification reaction reagents (such as cDNA PCR reaction reagents) in a container along with an appropriate mixture of nucleic acid primers; reacting the contents of the container to produce amplification products; and analyzing the amplification products to detect the presence of one or more ps20 polynucleotides in the sample. For mRNA the analyzing step may be accomplished using Northern Blot analysis to detect the presence of ps20 polynucleotides. The analysis step may be further accomplished by quantitatively detecting the presence of ps20 polynucleotides in the amplification product, and comparing the quantity of ps20 polynucleotides detected against a panel of expected values for the known presence or absence of the ps20 polynucleotides in normal samples derived using similar primers.

Therefore, disclosed is a method wherein MRNA is detected by (a) isolating mRNA from a sample and combining the mRNA with reagents to convert it to DNA; (b) treating the converted cDNA with amplification reaction reagents and nucleic acid primers that hybridize to one or more, ps20 polynucleotides to produce amplification products; (d) analyzing the amplification products , to detect an amount of mRNA encoding the ps20 polypeptides; and (e) comparing the amount of mR\A to an amount detected against a panel of expected values for normal and diseased samples derived using similar nucleic acid primers.

Disclosed is also a method comprising administering to cells or tissues imaging agents that carry labels for imaging and bind to ps20 polypeptides and optionally other markers of a viral disease, and then imaging the cells or tissues. Disclosed is an *in vivo* method comprising administering to a subject an agent that has been constructed to target one or more ps20 polypeptides.

Also disclosed are kits for carrying out the methods. In an aspect, the kit is for diagnosing a viral disease in a subject. In an example the kit is used for assessing whether a patient is afflicted with or has a pre-disposition to a viral disease and it comprises reagents for assessing one or more ps20 polypeptides or ps20 polynucleotides.

The methods, compositions, and kits described herein may be used with additional markers associated with a viral disease. Therefore, disclosed is a method for analyzing a biological sample for the presence of ps20 polypeptides and ps20 polynucleotides, and other markers that are specific indicators of a viral disease. The methods described herein may be modified by including reagents to detect the additional markers, or nucleic acids for the additional markers.

Disclosed is also a diagnostic composition comprising a ps20 polypeptide(s) or a ps20 polynucleotide(s). A composition is also provided comprising a probe that specifically hybridizes to a ps20 polynucleotide(s), or a fragment thereof, or an antibody specific for a ps20 polypeptide(s) or a fragment thereof. In another aspect, a composition is provided comprising one or more ps20 polynucleotide specific primer pairs capable of amplifying the polynucleotides using polymerase chain reaction methodologies. The probes, primers or antibodies can be labeled with a detectable substance.

Other objects, features and advantages of the present invention will become apparent from the following detailed description. It should be understood, however, that the detailed description and the specific examples while indicating preferred embodiments of the invention are given by way of illustration only.

### DESCRIPTION OF THE DRAWING

The invention will now be described in relation to the drawings in which:

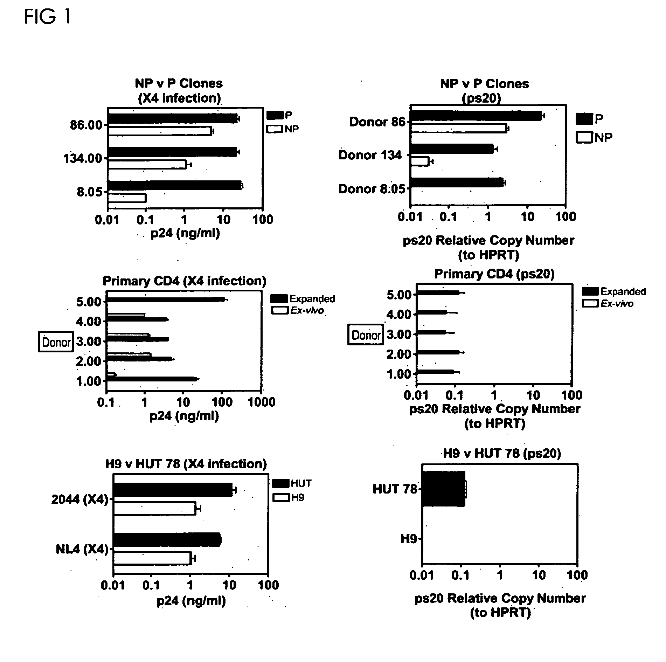

Figure 1 shows ps20 mRNA expression is associated with increased HIV spread: Comparison of H9 v HUT 78 immortalized cells infected with NL4-3 or 2044 strains. 1 million cells (clones, primacy CD4 CD45RO & H9/HUT 78) were infected overnight with virus dose standardised on HIV-Gag p24-CA concentration (2ng 2044 or 4ng NL4-3 strains). Expanded CD4s and clones were stimulated with allogeneic APC/PHA/IL2 for 5 days prior infection and cultured at 2x105/ml in 30IU/mlIL2 post infection. Mean peak p24-CA levels (day 8) in three biological replicate cultures is shown. qRT-PCR for ps20 per population was determined in triplicate at the time of infection and mean ps20 relative copy number (to HPRT) shown. **(a)** Comparison of non-permissive (NP) v permissive (P) counterpart clones from donors 8, 134 and 86 with 2044 (X4) strain. ***(b)***
   *Ex vivo:* CD4 CD45RO+ T-cells were infected with 2044, then cultured in 30IU/ml IL2. Expanded: Purified CD4+ CD45RO+ T-cells were expanded by two rounds of stimulation with allogeneic PBMC/PHA/IL2 (see M&M), then infected with 2044 and maintained in 30IU/ml IL2 post infection.

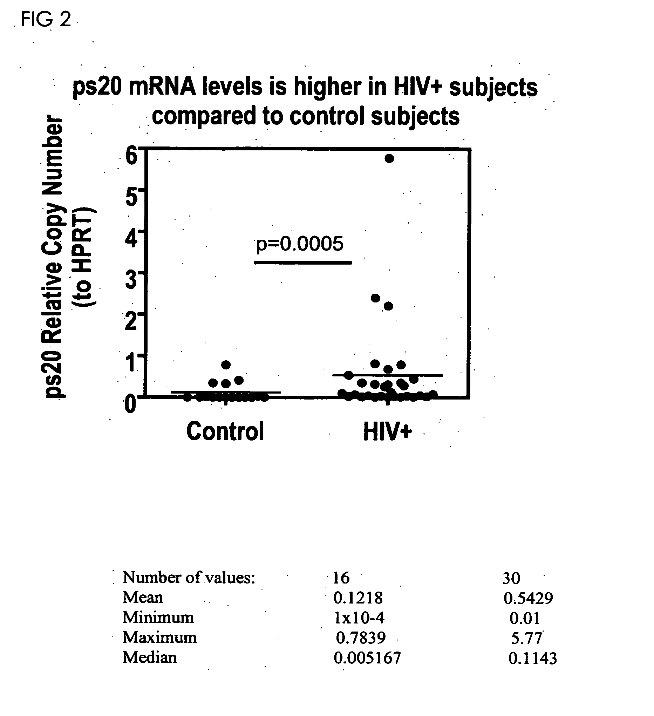

Figure 2 shows a plot. One million CD4 CD45RO+ T-cells were isolated from peripheral blood fractions of eight healthy volunteers and nine HIV+ chronically-infected subjects. Cells were activated with anti-CD3/28 Miltenyi as per manufacturers instructions) and 60IU/ml IL2 for 48 hours, RNA extracted and reverse transcribed. PCR was conducted with Qiagen primers for ps20 relative to a house keeping gene. (HPRT). Each sample was tested in 3-6 replicate wells depending on sample availability. Data shows the relative copy number of ps20 to a house keeping gene, HPRT. Data was analysed by Graph-Pad PRISM and P-value calculated by Wilcoxon Signed Rank Test shown
Figure 3 shows a bar chart and two graphs showing assays to measure ps20.
Figure 4 shows bar charts and a graph showing ps20 mRNA levels correlate with in vitro HIV infection.
Figure 5 shows bar charts showing ps20 mRNA is elevated in HIV infection in vivo

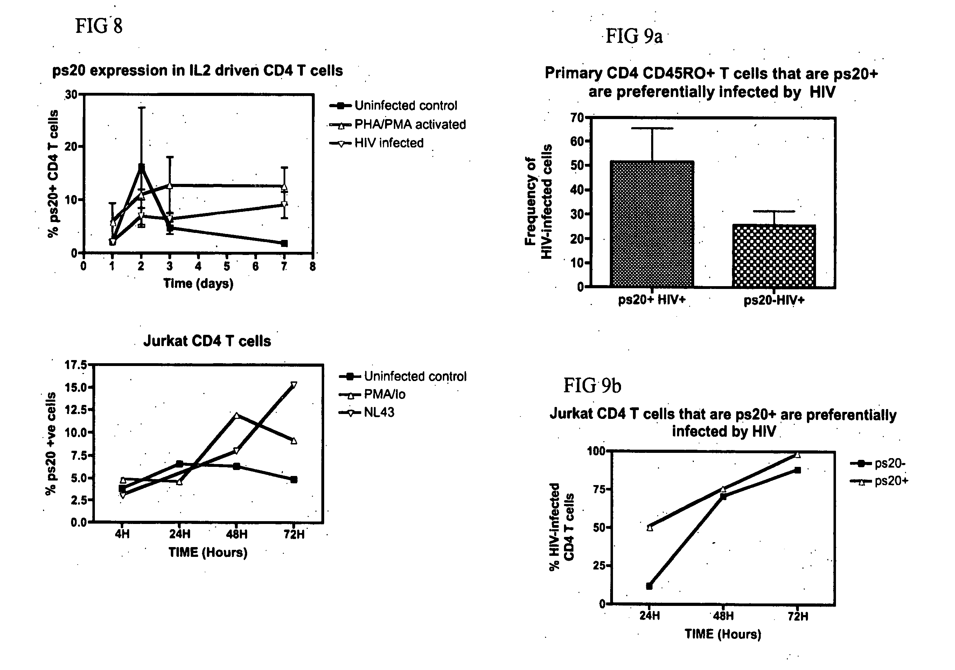

Figure 6 shows plots showing ps20 protein is elevated in plasma of HIV-infected subjects. Figure 7 shows graphs showng in vitro T cell activation up-regulates cell-surface ps20. Figure 8 shows graphs showing in vitro HIV infection up-regulates ps20
Figure 9 shows a bar chart and a graph showing ps20+ are preferentially susceptible to HIV infection compared to ps20- cells: therefore ps20 could be a marker of those cells that are most vulnerable to HIV infection

### DETAILED DESCRIPTION OF THE INVENTION

Methods are provided for characterizing or detecting the presence of a viral disease in a sample from a subject, the absence of a viral disease in a sample from a subject, the stage of a viral disease, and other characteristics of viral diseases that are relevant to prevention, diagnosis, characterization, and therapy of viral diseases in a subject. Methods are also provided for assessing the efficacy of a therapy for a viral disease, monitoring the progression of a viral disease, and selecting an agent or therapy for inhibiting a viral disease.

### Glossary

In accordance with the present invention there may be employed conventional biochemistry, enzymology, molecular biology, microbiology, and recombinant DNA techniques within the skill of the art. Such techniques are explained fully in the literature. See for example, Sambrook et al, Molecular Cloning: A Laboratory Manual, Third Edition (2001) Cold Spring Harbor Laboratory Press, Cold Spring Harbor, N.Y); DNA Cloning: A Practical Approach, Volumes I and II (D.N. Glover ed. 1985); Oligonucleotide Synthesis (M.J. Gait ed. 1984); Nucleic Acid Hybridization B.D. Hames & S.J. Higgins eds. (1985); Transcription and Translation B.D. Hames & S.J. Higgins eds (1984); Animal Cell Culture R.I. Freshney, ed. (1986); Immobilized Cells and enzymes IRL Press, (1986); and B. Perbal, A Practical Guide to Molecular Cloning (1984).

Unless defined otherwise, all technical and scientific terms used herein have the same meaning as commonly understood by one of ordinary skill in the art to which this invention belongs.

The recitation of numerical ranges by endpoints herein includes all numbers and fractions subsumed within that range (e.g. 1 to 5 includes 1, 1.5, 2, 2.75, 3, 3.90, 4, and 5). It is also to be understood that all numbers and fractions thereof are presumed to be modified by the term "about." Further, it is to be understood that "a," "an," and "the" include plural referents unless the content clearly dictates otherwise. The term "about" means plus or minus 0.1 to 50%, 5-50%, or 10-40%, preferably. 10-20%, more preferably 10% or 15%, of the number to which reference is being made.

The terms "peptide", "polypeptide" and "protein" are used interchangeably and as used herein refer to more than one amino acid joined by a peptide bond.

"Synthetic" refers to items, e.g., polypeptides or polynucleotides, which are not naturally occurring, in that they are isolated, synthesized or otherwise manipulated by man.

"Composition" includes any composition of matter, including peptides, polypeptides, proteins, mixtures, antibodies, or markers of the present invention.

"Detectable substances" include, but are not limited to, the following: radioisotopes (e.g., ³H, ¹⁴C, ³⁵S, ¹²⁵I, ¹³¹I), fluorescent labels (e.g., FITC, rhodamine, lanthanide phosphors), luminescent labels such as luminol, enzymatic labels (e.g., horseradish peroxidase, beta-galactosidase, luciferase, alkaline phosphatase, acetylcholinesterase), biotinyl groups (which can be detected by marked avidin e.g., streptavidin containing a fluorescent marker or enzymatic activity that can be detected by optical or colorimetric methods), and predetermined polypeptide epitopes recognized by a secondary reporter (e.g., leucine zipper pair sequences, binding sites for secondary antibodies, metal binding domains, epitope tags).

"Viral diseases" means a class of diverse diseases and disorders caused by or believed to be caused by viruses. The term includes any stage of a viral infection, including incubation phase; latent or dormant phase, acute phase, and development and maintenance of immunity towards a virus. Consequently, the term "treatment' is meant to include aspects of generating or restoring immunity of the patient's immune system, at well as aspects of suppressing or inhibiting virus activity. "Virus activity" includes virus replication, assembly, maturation, envelopment, extracellular virus formation, virus egress, and virus transmission. Viral diseases include, without limitation, genital warts (HPV), HIV/AIDS, herpes, influenza, measles, polio, varicella-zoster, hepatitis A, hepatitis B, hepatitis C, hepatitis D, herpes simplex virus (type 1 and type 2), hepatitis E, hepatitis G, cytomegalovirus, meningitis, genital warts (HPV), a disease associated with respiratory syncytial virus infection, a disease associated with coxsackie virus infection, a disease associated with ebola virus infection, a disease associated with hantavirus infection, a disease associated with human papilloma virus infection, a disease associated with rotavirus infection, a disease associated with west nile virus infection, a disease associated with Epstein-Barr virus infection, a disease associated with papilloma virus infection, a disease associated with influenza virus infection, vesticular stomatitis virus infection, and dengue fever. The clinical sequelae of viral infections include without limitation, herpes, AIDS, lassa fever, kaposi's sarcoma, meningitis, mumps, polio, chicken pox, colds and flu, dengue fever, encephalitis, Fifth disease, shingles, genital warts, rubella, yellow fever, hepatitis A, B and C, measles, rabies, and smallpox. The singular form "viral disease" includes any one or more diseases selected from the class of viral diseases, and includes any compound or complex disease state wherein a component of the disease state includes a disease selected from the class of viral diseases.

The terms "sample", "biological sample", and the like mean a material known or suspected of expressing or containing ps20 polypeptides or ps20 polynucleotides. A test sample can be used directly as obtained from the source or following a pretreatment to modify the character of the sample. The sample can be derived from any biological source, such as tissues, extracts, or cell cultures, including cells (e.g. T cells, in particular CD4 T cells), cell lysates, and physiological fluids, such as, for example, whole blood, plasma, serum, saliva, ocular lens fluid, cerebral spinal fluid, sweat, urine, milk, ascites fluid, synovial fluid, peritoneal fluid, lavage fluid, and the like. The sample can be obtained from animals, preferably mammals, most preferably humans. The sample can be treated prior to use, such as preparing plasma from blood, diluting viscous fluids, and the like. Methods of treatment can involve filtration, distillation, extraction, concentration, inactivation of interfering components, the addition of reagents, and the like.

In an embodiment the sample its a human physiological fluid. In a particular embodiment, the sample is human serum. In a more particular embodiment, the sample comprises T cells, most particularly CD4 T cells.

The terms "subject" or "patient" refer to an animal including a warm-blooded animal such as a mammal, which is afflicted with or suspected of having or being predisposed to a viral disease. Mammal includes without limitation any members of the Mammalia. In general, the terms refer to a human. The terms also include animals bred , for food, sport, or as pets, including domestic animals such as horses, cows, sheep, poultry, fish, pigs, and goats, and cats; dogs, and zoo animals, apes (e.g. gorilla or chimpanzee), and rodents such as rats and mice.

The term "ps20 polypeptide" includes human ps20, in particular the native-sequence polypeptide, isoforms, chimeric polypeptides, all homologs, fragments, precursors, complexes, and modified forms and derivatives of human ps20. The amino acid sequence for native human ps20 includes the sequences of Accession Nos. NP_067020, EAW95486, EAW95487, AAG16647, AAG15263.1, Q9HC57, BAC11377.1, ABM84291.1, or ABM87681.1 or shown in SEQ ID NO. 2 and 3.

A "native-sequence polypeptide" comprises a polypeptide having the same amino acid sequence of a polypeptide derived from nature. Such native-sequence polypeptides can be isolated from nature or can be produced by recombinant or synthetic means. The term specifically encompasses naturally occurring truncated or secreted forms of a polypeptide, polypeptide variants including naturally occurring variant forms (e.g. alternatively spliced forms or splice variants), and naturally occurring allelic variants.

The term "polypeptide variant" means a polypeptide having substantial sequence identity. In an aspect a polypeptide variant has at least about 45%, preferably at least about 85%, more preferably at least about 90%, most preferably at least about 95% amino acid sequence identity with a native-sequence polypeptide. Polypeptide variants preferably retain the immunogenic activity of a corresponding native-sequence polypeptide. Particular polypeptide variants have at least 45%, preferably 50%, 55%, 60%, 65%, 70%, 75%, 80%, 85%, 90%, 95%, 97%, 98%, or 99% sequence identity to the sequences identified in Accession Nos. NP 067020, EAW95486, EAW95487, AAG16647, AAG15263.1, Q9HC57, BAC11377.1, ABM84291.1, or ABM87681.1, or shown in SEQ, ID NO. 2 and 3. Polypeptide variants also include, for instance, polypeptides wherein one or more amino acid residues are added to, or deleted from, the N- or C-terminus of the full-length or mature sequences of the polypeptide, including variants from other species, but excludes a native-sequence polypeptide.

Percent identity of two amino acid sequences, or of two nucleic acid sequences is defined as the percentage of amino acid residues or nucleotides in a candidate sequence that are identical with the amino acid residues in a polypeptide or nucleic acid sequence, after aligning the sequences and introducing gaps, if necessary, to achieve the maximum percent sequence identity, and not considering any conservative substitutions as part of the sequence identity. Alignment for purposes of determining percent amino acid or nucleic acid sequence identity can be achieved in various conventional ways, for instance, using publicly available computer software including the GCG program package (Devereux J. et al., .Nucleic Acids Research 12(1): 387, 1984); BLASTP, BLASTN, and FASTA (Atschul, S.F. et al. J.Molec. Biol. 215: 403-410, 1990). The BLAST X program is publicly available from NCBI and other sources (BLAST Manual, Altschul, S. et al. NCBI NLM NIH Bethesda, Md. 20894; Altschul, S. et al. J. Mol. Biol. 215: 403-410, 1990). Skilled artisans can determine appropriate parameters for measuring alignment, including any algorithms needed to achieve maximal alignment over the full length of the sequences being compared. Methods to determine identity and similarity are codified in publicly available computer programs.

A variant may also be created by introducing substitutions, additions, or deletions into a polynucleotide encoding a native polypeptide sequence such that one or more amino acid substitutions, additions, or deletions are introduced into the encoded protein. Mutations may be introduced by standard methods, such as site-directed mutagenesis and PCR-mediated mutagenesis. In an embodiment, conservative substitutions are made at one or more predicted non-essential amino acid residues. A "conservative amino acid substitution" is one in which an amino acid residue is replaced with an amino acid residue with a similar side chain. Amino acids with similar side chains are known in the art and include amino acids with basic side chains (e.g. Lys, Arg, His), acidic side chains (e.g. Asp, Glu), uncharged polar side chains (e.g. Gly, Asp, Glu, Ser, Thr, Tyr and Cys), nonpolar side chains (e.g. Ala, Val, Leu, Iso, Pro, Trp), beta-branched side chains (e.g. Thr, Val, Iso), and aromatic side chains (e.g. Tyr, Phe, Trp, His). Mutations can also be introduced randomly along part or all of the native sequence, for example, by saturation mutagenesis. Following mutagenesis the variant polypeptide can be recombinantly expressed and the activity of the polypeptide may be determined.

Polypeptide, variants include polypeptides comprising amino acid sequences sufficiently identical to or derived from the amino acid sequence of a native polypeptide which include fewer amino acids than the full length polypeptides. A portion of a polypeptide can be a polypeptide which is for example, 10, 15, 20, 25, 30, 35, 40, 45, 50, 60, 70, 80, 90, 100 or more amino acids in length. Portions in which regions of a polypeptide are deleted can be prepared by recombinant techniques and can be evaluated for one or more functional activities such as the ability to form antibodies specific for a polypeptide.

A naturally occurring allelic variant may contain conservative amino acid substitutions from the native polypeptide sequence or it may contain a substitution of an amino acid from a corresponding position in a polypeptide homolog, for example, a murine polypeptide.

Ps20 polypeptides include chimeric or fusion proteins. A "chimeric protein" or "fusion protein" comprises all or part (preferably biologically active) of ps20 polypeptide operably linked to a heterologous polypeptide (i.e., a polypeptide other than a ps20 polypeptide). Within the fusion protein, the term "operably linked" is intended two indicates that a ps20 polypeptide and the heterologous polypeptide are fused in-frame to each other. The heterologous polypeptide can be fused to the N-terminus or C-terminus of a ps20 polypeptide. A useful fusion protein is a GST fusion protein in which a ps20 polypeptide is fused to the C-terminus of GST sequences. Another example of a fusion protein is an immunoglobulin fusion protein in which all or part of a ps20 polypeptide is fused to sequences derived from a member of the immunoglobulin protein family. Chimeric and fusion proteins can be produced by standard recombinant DNA techniques.

A modified form of a polypeptide referenced herein includes modified forms of the polypeptides and derivatives of the polypeptides, including but not limited to glycosylated, phosphorylated, acetylated, methylated or lapidated forms of the polypeptides. For example, an N-terminal methionine may be cleaved from as polypeptide, and a new N-terminal residue may or may not be acetylated.

Ps20 polypeptides may be prepared by recombinant or synthetic methods, or isolated from a variety of sources, or by any combination of these and similar techniques.

"Ps20 polynucleotide(s)" refers to polynucleotides encoding ps20 polypeptides including native-sequence polypeptides, polypeptide variants including a portion of a polypeptide, an isoform, precursor, complex, a chimeric polypeptide, or modified forms and derivatives of the polypeptides. A polynucleotide encoding a native polypeptide employed in the present invention includes the polynucleotides encoding ps20 [e.g., Accession Nos. NM_021197, AF169631, AAG16647.1, AF302109, AAG15263.1, AK075061, BAC11377.1; BC029159, AAH29159.1, AC010551.3, CH471114.2, AL713785, AL713785, CR595501, CR604862, CR608359, CR610530, CR615719, DQ893365.2, or DQ896682.2, Gene ID No. 58189, or SEQ ID NO. 1].

Ps20 polynucleotides include complementary nucleic acid sequences, and nucleic acids that are substantially identical to these sequences (e.g. at least about 45%, preferably 50%, 55%, 60%, 65%, 70%, 75%, 80%, 85%, 90%, 95%, 97%, 98%, or 99% sequence identity).

Ps20 polynucleotide further include sequences that differ from a native sequence [e.g. Accession Nos. NM_021197, AF169631, AAG16647.1, AF302109, AAG15263.1, AK075061, BACI1377.1, BC029159, AAH29159.1, AC010551.3, CH471114.2, AL713785, AL713785, CR595501, CR604862, CR608359, CR610530, CR615719, Do893365.2; or .do896682.2, Gene ID No. 58189, or SEQ ID NO. 1] due to degeneracy in the genetic code. As one example, DNA sequence polymorphisms within the nucleotide sequence of a ps20 polypeptide may result in silent mutations that do not affect the amino acid sequence. Variations in one or more nucleotides may exist among individuals within a population due to natural allelic variation. DNA sequence polymorphisms may also occur which lead to changes in the amino acid sequence of a polypeptide.

Ps20 polynucleotides also include nucleic acids that hybridize under stringent conditions, preferably high stringency conditions to a ps20 polynucleotide [e.g. Accession Nos. NM_021197, AF169631, AAG16647.1, AF302109, AAG15263.1, AK075061, BAC11377.1, BC029159, AAH29159.1, AC010551.3, CH471114.2, AL713785, AL713785, CR595501, CR604862, CR608359, CR610530, CR615719, DQ893365.2, or DQ896682.2, Gene ID No. 58189, or SEQ ID NO. 1]. Appropriate stringency conditions which promote DNA hybridization are known to those skilled in the art, or can be found in Current Protocols in Molecular Biology, John Wiley & Sons, N.Y..(1989), 6.3.1-6.3.6. For example, 6.0 x sodium chloride/sodium citrate (SSC) at about 45°C, followed by a wash of 2.0 x SSC at 50°C may be employed. The stringency may be selected based on the conditions used in the wash step. By way of example, the salt concentration in the wash step can be selected from a high stringency of about 0.2 x SSC at 50°C. In addition, the temperature in the wash step can be at high stringency conditions, at about 65°C.

Ps20 polynucleotides also include truncated nucleic acids or nucleic acid fragments and variant forms of the nucleic acids that arise by alternative splicing of an mRNA corresponding to a DNA.

The ps20 polynucleotides are intended to include DNA and RNA (e.g. mRNA) and can be either double stranded or single stranded. A polynucleotide may, but need not, include additional coding or non-coding sequences, or it may, but need not, be linked to other molecules and/or carrier or support materials. The polynucleotides for use in the methods of the invention may be of any length suitable for a particular method. In certain applications the term refers to antisense polynucleotides (e.g. mRNA or DNA strand in the reverse orientation to sense ps20 polynucleotides).

A "significant difference" in levels of ps20 polypeptides or polypeptides in a sample compared to a control or standard (e.g. normal levels or levels in other samples from a subject) may represent levels that are higher or lower than the standard error of the detection assay. In particular embodiments, the levels may be 1.5, 2, 3, 4, 5, or 6 times higher or lower than the control or standard.

"Binding agent" refers to a substance that specifically binds to one or more ps20 polypeptides. A substance "specifically binds" to one or more ps20 polypeptides if reacts at a detectable level with one or more ps20 polypeptides, and does not react detectably with peptides containing an unrelated or different sequence. Binding properties may be assessed using an ELISA, which may be readily performed by those skilled in the art (see for example, Newton et al., Develop. Dynamics 197: 1-13, 1993).

A binding agent may be a ribosome, with or without a peptide component, an aptamer, an RNA molecule, or a polypeptide. A binding agent may be a polypeptide that comprises one or more ps20 polypeptide sequence, a peptide variant thereof, or a non-peptide mimetic of such a sequence. By way of example, a ps20 polypeptide sequence may be a peptide portion of a ps20 polypeptide that is capable of modulating a function mediated by a ps20 polypeptide.

An aptamer includes a DNA or RNA molecule that binds to nucleic acids and proteins. An aptamer that binds to a protein (or binding domain) of a ps20 polypeptide or a ps20 polynucleotide can be produced using conventional techniques, without undue experimentation. [For example, see the following publications describing *in vitro* selection of aptamers: Klug et al., Mol. Biol. Reports 20:97-107 (1994); Wallis et al., Chem. Biol. 2:543-552 (1995); Ellington, Curr. Biol. 4:427-429 (1994); Lato et al., Chem. Biol. 2:291-303 (1995); Conrad et al., Mol. Div. 1:69-78 (1995); and Uphoff et al., Curr. Opin. Struct. Biol. 6:281-287 (1996)].

Antibodies for use in the present invention include but are not limited to monoclonal or polyclonal antibodies, immunologically active fragments (e.g. a Fab or (Fab)₂ fragments), antibody heavy chains, humanized antibodies, antibody light chains, genetically engineered single chain Fᵥ molecules (Ladner et al, U.S. Pat. No. 4,946,778), chimeric antibodies, for example, antibodies which contain the binding specificity of murine antibodies, but in which the remaining portions are of human origin, or derivatives, such as enzyme conjugates or labeled derivatives.

In an embodiment of the invention, antibodies are reactive against or specific for ps20 polypeptides if they bind with a Kₐ of greater than or equal to 10⁻⁷ M.

Antibodies including monoclonal and polyclonal antibodies, fragments and chimeras, may be prepared using methods known to those skilled in the art. Isolated native or recombinant ps20 polypeptides may be utilized to prepare antibodies. See, for example, Kohler et al. (1975) Nature 256:495-497; Kozbor et al. (1985) J. Immunol Methods 81:31-42; Cote et al. (1983) Proc Natl Acad Sci 80:2026-2030; and Cole et al. (1984) Mol Cell Biol 62:109-120 for the preparation of monoclonal antibodies; Huse et al. (1989) Science 246:1275-1281 for the preparation of monoclonal Fab fragments; and, Pound (1998) Immunochemical Protocols, Humana Press, Totowa, N.J., for the preparation of phagemid or B-lymphocyte immunoglobulin libraries to identify antibodies. Antibodies specific for ps20 polypeptides may also be obtained from scientific or commercial sources.

"Microarray" and "array," refer to nucleic acid or nucleotide arrays or protein or peptide arrays that can be used to detect biomolecules associated with a viral disease, for instance to measure gene expression. A variety of arrays are made in research and manufacturing facilities worldwide, some of which are available commercially. By way of example, spotted arrays and *in situ* synthesized arrays are two kinds of nucleic acid arrays that differ in the manner in (which the nucleic acid materials are placed onto the array substrate. A widely used *in situ* synthesized oligonucleotide array is GeneChip™ made by Affymetrix, Inc. Oligonucleotide probes that are 20- or 25-bases long can be synthesized *in silico* on the array substrate. These arrays can achieve high densities (e.g., more than 40,000 genes per cm²). Generally spotted arrays have lower densities, but the probes, typically partial cDNA molecules, are much longer than 20- or 25-mers. Examples of spotted cDNA arrays include LifeArray made by Incyte Genomics and DermArray made by IntegriDerm (or Invitrogen). Pre-synthesized and amplified cDNA sequences are attached to the substrate of spotted arrays. Protein and peptide arrays also are known (see for example, Zhu et al., Science 293:2101 (2001).

### Markers

The invention provides markers correlated with viral diseases. A set of these markers identified as useful for detection, diagnosis, and prevention of viral disease comprises one or more ps20 polypeptides and/or ps20 polynucleotides. The invention provides a ps20 marker set that distinguishs viral disease and uses therefore comprising or consisting of one or more ps20 polypeptides and/or ps20 polynucleotides. Disclosed is a method for classifying a viral disease comprising detecting a difference in the expression of ps20 markers relative to a control, the ps20 markers consisting of one or more ps20 polypeptides and/or ps20 polynucleotides. In an aspect, the control comprises markers derived from a pool of samples from individual patients with no viral disease. Any of the ps20 markers provided herein may be used alone or with other markers of viral disease, or with markers for other phenotypes or conditions.

### Nucleic Acid Methods/Assays

As noted herein a viral disease may be diagnosed or detected based on the amounts/levels of ps20 polynucleotides in a sample. Techniques for detecting polynucleotides such as polymerase chain reaction (PCR) and hybridization assays are well known in the art.

Probes may be used in hybridization techniques to detect ps20 polynucleotides. The technique generally involves contacting and incubating nucleic acids (e.g. recombinant DNA molecules, cloned genes) obtained from a sample from a patient or other cellular source with a probe under conditions favorable for the specific annealing of the probes to complementary sequences in the nucleic acids. After incubation, the non-annealed nucleic acids are removed, and the presence of nucleic acids that have hybridized to the probe if any are detected.

Nucleotide probes for use in the detection of nucleic acid sequences in samples may be constructed using conventional methods known in the art. Suitable probes may be based on nucleic acid sequences encoding at least 5 sequential amino acids from regions of ps20 polynucleotides, preferably they comprise 10-150, more particularly 10-30, 10-40, 20-50, 40-80, 50-150, 80-120 nucleotides in length. The probes may comprise DNA or DNA mimics (e.g., derivatives and analogues) corresponding to a portion of an organism's genome, or complementary RNA or RNA mimics. Mimics are polymers comprising subunits capable of specific, Watson-Crick-like hybridization with DNA, or of specific hybridization with RNA. The nucleic acids can be modified at the base moiety, at the sugar moiety, or at the phosphate backbone.

DNA can be obtained using standard methods such as polymerase chain reaction (PCR) amplification of genomic DNA or cloned sequences. (See, for example, in Innis et al., eds., 1990, PCR Protocols: A Guide to Methods and Applications, Academic Press Inc., San Diego, Calif.). Computer programs known in the art can be used to design primers with the required specificity and optimal amplification properties, such as Oligo version 5.0 (National Biosciences). Controlled robotic systems may be useful for isolating and amplifying nucleic acids.

A nucleotide probe may be labeled with a detectable substance such as a radioactive label that provides for an adequate signal and has sufficient half-life such as ³²P, ³H, ¹⁴C or the like. Other detectable substances that may be used include antigens that are recognized by a specific labeled antibody, fluorescent compounds, enzymes, antibodies specific for a labeled antigen, and luminescent compounds. An appropriate label may be selected having regard to the rate of hybridization and binding of the probe to the nucleotide to be detected and the amount of nucleotide available for hybridization. Labeled probes may be hybridized to nucleic acids on solid supports such as nitrocellulose filters or nylon membranes as generally described in Sambrook et al, 1989, Molecular Cloning, A Laboratory Manual (2nd ed.). The nucleic acid probes may be used to detect ps20 polynucleotides. The nucleotide probes may also be useful in the diagnosis of a viral disease involving one or more ps20 polynucleotides, in monitoring the progression of such disorder, or monitoring a therapeutic treatment.

The detection of ps20 polynucleotides may involve the amplification of specific gene sequences using an amplification method such as polymerase chain reaction (PCR), followed by the analysis of the amplified molecules using techniques known to those skilled in the art. Suitable primers can be routinely designed by one of skill in the art. By way of example, at least two oligonucleotide primers may be employed in a PCR based assay to amplify a portion of a polynucleotide encoding one or more ps20 polynucleotides derived from a sample, wherein at least one of the oligonucleotide primers is specific for (i.e. hybridizes to) a polynucleotide encoding a ps20 polypeptide. Examples of primers are described in Example 1. The amplified cDNA is then separated and detected using techniques well known in the art, such as gel electrophoresis.

In order to maximize hybridization under assay conditions, primers and probes employed in the methods of the invention generally have at least about 60%, preferably at least about 75%, and more preferably at least about 90% identity to a portion of a ps20 polynucleotide; that is, they are at least 10 nucleotides, and preferably at least 20 nucleotides in length. In an embodiment the primers and probes are at least about 10-40 nucleotides in length.

Hybridization and amplification techniques described herein may be used to assay qualitative and quantitative aspects of ps20 polynucleotide expression. For example, RNA may be isolated from a cell type known to express a ps20 polynucleotide (e.g., CD4 T cells) and tested utilizing the hybridization (e.g. standard Northern analyses) or PCR techniques referred to herein.

In an example, a method is provided employing reverse transcriptase-polymerase chain reaction (RT-PCR), in which PCR is applied in combination with reverse transcription. Generally, RNA is extracted from a sample tissue using standard techniques (for example, guanidine isothiocyanate extraction as described by Chomcynski and Sacchi, Anal. Biochem. 162:156-159, 1987) and is reverse transcribed to produce cDNA. The cDNA is used as a template for a polymerase chain reaction. The cDNA is hybridized to a set of primers, at least one of which is specifically designed against a ps20 polynucleotide sequence. Once the primer and template have annealed a DNA polymerase is employed to extend from the primer, to synthesize a copy of the template. The DNA strands are denatured, and the procedure is repeated many times until sufficient DNA is generated to allow visualization by for example, ethidium bromide staining and agarose gel electrophoresis.

Amplification may be performed on samples obtained from a subject with a suspected viral disease and an individual who is not afflicted with a viral disease. The reaction may be performed on several dilutions of cDNA spanning at least two orders of magnitude. A significant difference in expression in several dilutions of the subject sample as compared to the same dilutions of the non-disease sample may be considered positive for the presence of a viral disease.

In an example, disclosed herein are methods for determining the presence or absence of a viral disease in a subject comprising (a) contacting a sample obtained from the subject with oligonucleotides that hybridize to ps20 polynucleotides; and (b) detecting in the sample a level of nucleic acids that hybridize to the polynucleotides relative to a predetermined cut-off value, and therefrom determining the presence or absence of a viral disease in the subject.

Disclosed is a method wherein a ps20 polynucleotide mRNA is detected by (a) isolating mRNA from a sample and combining the mRNA with reagents to convert it to cDNA; (b) treating the converted cDNA with amplification reaction reagents and nucleic acid primers that hybridize to one or more ps20 polynucleotides to produce amplification products; (d) analyzing the amplification products to detect amounts of mRNA encoding ps20 polypeptides; and (e) comparing the amount of mRNA to an amount detected against a panel of expected values for normal subjects derived using similar nucleic acid primers. Ps20 polynucleotide-positive samples or alternatively higher levels in patients compared to a control (e.g. non-infected individual) may be indicative of viral disease, advanced viral disease, and/or that the patient is not responsive to therapy.

In another example, disclosed are methods for diagnosing or determining the presence or absence of a viral disease in a subject comprising (a) contacting a sample obtained from the subject with oligonucleotides that hybridize to one or more ps20. polynucleotides; and (b) detecting in the sample levels of nucleic acids that hybridize to the polynucleotides relative to a predetermined cut-off value, and therefrom determining the presence or absence of a viral disease in the subject. In particular, disclosed is a method wherein *WFDC1* mRNA is detected by (a) isolating mRNA from a sample and combining the mRNA with reagents to convert it to cDNA; (b) treating the converted cDNA with amplification reaction reagents and nucleic acid primers that hybridize to *WFDC1* to produce amplification products; (d) analyzing the amplification products to detect an amount of *WFDC1* mRNA; and (e) comparing the amount of mRNA to an amount detected against a panel of expected values for healthy individuals derived using similar nucleic acid primers. *WFDC1*-positive samples or alternatively higher levels, in particular significantly higher levels of *WFDC1* in patients compared to a control (e.g. normal) are indicative of a viral disease.

Oligonucleotides or longer fragments derived from ps20 polynucleotides may be used as targets in a microarray. The microarray can be used to simultaneously monitor the expression levels of large numbers of genes and to identify genetic variants and mutations. The information from the microarray may be used to determine gene function, to diagnose a viral disease, and to develop and monitor the activities of therapeutic agents. The preparation, use, and analysis of microarrays are well known to a person skilled in the art. (See, for example, Brennan, T. M. et al. (1995) U.S. Pat. No. 5,474,796; Schena, et al. (1996) Proc. Natl. Acad. Sci. 93:10614-10619; Baldeschweiler et al. (1995), PCT Application WO95/251116; Shalon, D. et al. (1995) PCT application WO95/35505; Heller, R. A. et al. (1997) Proc. Natl. Acad. Sci. 94:2150-2155; and Heller, M. J. et al. (1997) U.S. Pat. No. 5,605,662.)

Thus, disclosed is also includes an array comprising one or more ps20 polynucleotides and optionally other markers. The array can be used to assay expression of ps20 polynucleotides in the array. The invention allows the quantitation of expression of one or more ps20 polynucleotides.

Microarrays typically contain at separate sites nanomolar quantities of individual genes, cDNAs, or ESTs on a substrate (e.g.nitrocellulose or silicon plate), or photolithographically prepared glass substrate. The arrays are hybridized to cDNA probes using conventional techniques with gene-specific primer mixes. The target polynucleotides to be analyzed are isolated, amplified and labelled, typically with fluorescent labels, radiolabels or phosphorous label probes. After hybridization is completed, the array is inserted into the scanner, where patterns of hybridization are detected. Data are collected as light emitted from the labels incorporated into the target, which becomes bound to the probe array. Probes that completely match the target generally produce stronger signals than those that have mismatches. The sequence and position of each probe on the array are known, and thus by complementarity, the identity of the target nucleic acid applied to the probe array can be determined.

Microarrays are prepared by selecting polynucleotide probes and immobilizing them to a solid support or surface. The probes may comprise. DNA sequences, RNA sequences, copolymer sequences of DNA and RNA, DNA and/or RNA analogues, or combinations thereof. The probe sequences may be full or partial fragments of genomic DNA, or they may be synthetic oligonucleotide sequences synthesized either enzymatically in vivo, enzymatically *in vitro* (e.g., by PCR), or non-enzymatically *in vitro*.

The probe or probes used in methods of the invention can be immobilized to a solid support or surface which may be either porous or non-porous. For example, the probes can be attached to a nitrocellulose or nylon membrane or filter covalently at either the 3' or the 5' end of the polynucleotide probe. The solid support may be a glass or plastic surface. In an aspect of the invention, hybridization levels are measured to microarrays of probes consisting of a solid support on the surface of which are immobilized a population of polynucleotides, such as a population of DNA or DNA mimics, or, alternatively, a population of RNA or RNA mimics. A solid support may be a nonporous or, optionally, a porous material such as a gel.

In accordance with further examples a microarray is disclosed comprising a support or surface with an ordered array of hybridization sites or "probes" each representing one of the markers described herein. The microarrays can be addressable arrays, and in particular positionally addressable arrays. Each probe of the array is typically located at a known, predetermined position on the solid support such that the identity of each probe can be determined from its position in the array. In preferred embodiments, each probe is covalently attached to the solid support at a single site.

Microarrays used are preferably (a) reproducible, allowing multiple copies of a given array to be produced and easily compared with each other; (b) made from materials that are stable under hybridization conditions; (c) small, (e.g., between 1 cm² and 25 cm², between 12 cm² and 13 cm², or 3 cm²; and (d) comprise a unique set of binding sites that will specifically hybridize to the product of a single gene in a cell (e.g., to a specific mRNA, or to a specific cDNA derived therefrom). However, it will be appreciated that larger arrays may be used particularly in screening arrays, and other related or similar sequences will cross hybridize to a given binding site.

In accordance with an example, the microarray is an array in which each position represents one of the ps20 polynucleotides described herein. Each position of the array can comprise a DNA or DNA analogue based on genomic DNA to which a particular RNA or cDNA transcribed from a genetic marker can specifically hybridize. A DNA or DNA analogue can be a synthetic oligomer or a gene fragment.

Probes for the microarray can be synthesized using N-phosphonate or phosphoramidite chemistries (Froehler et al., 1986, Nucleic Acid Res. 14:5399-5407; McBride et al., 1983, Tetrahedron Lett. 24:246-248). Synthetic sequences are typically between about 10 and about 500 bases, 20-100 bases, or 40-70 bases in length. Synthetic nucleic acid probes can include non-natural bases, such as, without limitation, inosine. Nucleic acid analogues such as peptide nucleic acid may be used as binding sites for hybridization. (see, e.g., Egholm et al., 1993, Nature 363:566-568; U.S. Pat. No. 5,539,083).

Probes can be selected using an algorithm that takes into account binding energies, base composition, sequence complexity, cross-hybridization binding energies, and secondary structure (see Friend et al., International Patent Publication WO 01/05935, published Jan. 25, 2001).

Positive control probes, (e.g., probes known to be complementary and hybridize to sequences in the target polynucleotides), and negative control probes, (e.g., probes known to not be complementary and hybridize to sequences in the target polynucleotides) are typically included on the array. Positive controls can be synthesized along the perimeter of the array or synthesized in diagonal stripes across the array. A reverse complement for each probe can be next to the position of the probe to serve as a negative control.

The probes can be attached to a solid support or surface, which may be made from glass, plastic (e.g., polypropylene, nylon), polyacrylamide, nitrocellulose, gel, or other porous or nonporous material. The probes can be printed on surfaces such as glass plates (see Schena et al., 1995, Science 270:467-470). This method may be particularly useful for preparing microarrays of cDNA (See also, DeRisi et al., 1996, Nature Genetics 14:457-460; Shalon et al., 1996, Genome Res. 6:639-645; and Schena et al., 1995, Proc. Natl. Acad. Sci. U.S.A. 93:10539-11286).

High-density oligonucleotide arrays containing thousands of oligonucleotides complementary to defined sequences, at defined locations on a surface can be produced using photolithographic techniques for synthesis *in situ* (see, Fodor et al., 1991, Science 251:767-773; Pease et al., 1994, Proc. Natl. Acad. Sci. U.S.A. 91:5022-5026; Lockhart et al., 1996, Nature Biotechnology 14:1675; U.S. Pat. Nos. 5,578,832; 5,556,752; and 5,510,270) or other methods for rapid synthesis and deposition of defined oligonucleotides (Blanchard et al., Biosensors & Bioelectronics 11:687-690). Using these methods oligonucleotides (e.g., 60-mers) of known sequence are synthesized directly on a surface such as a derivatized glass slide. The array produced may be redundant, with several oligonucleotide molecules per RNA.

Microarrays can be made by other methods including masking (Maskos and Southern, 1992, Nuc. Acids. Res. 20:1679-1684). In an example, microarrays are produced by synthesizing polynucleotide probes on a support wherein the nucleotide probes are attached to the support covalently at either the 3' or the 5' end of the polynucleotide.

Disclosed herein are microarrays comprising a disclosed marker set. In one example, disclosed is a microarray for distinguishing viral disease samples comprising a positionally-addressable array of polynucleotide probes bound to a support, the polynucleotide probes comprising a plurality of polynucleotide probes of different nucleotide sequences, each of the different nucleotide sequences comprising a sequence complementary and hybridizable to a plurality of genes, the different nucleotide sequences comprising ps20 polynucleotides.

Disclosed are gene marker sets that distinguish viral disease and uses thereof. In an aspect, disclosed is a method for classifying a viral disease comprising detecting a difference in the expression of a first plurality of genes relative to a control, the first plurality of genes comprising ps20 polynucleotides. In another specific aspect, the control comprises nucleic acids derived from a pool of samples from individual control patients.

### Protein Methods

Binding agents may be used for a variety of diagnostic and assay applications to assay polypeptides in samples. There are a variety of assay formats known to the skilled artisan for using a binding agent to detect a target molecule in a sample. (For example, see Harlow and Lane, Antibodies: A Laboratory Manual, Cold Spring Harbor Laboratory, 1988). In general, the presence or absence of a viral disease in a subject may be determined by (a) contacting a sample from the subject with a binding agent; (b) detecting in the sample a level of a ps20 polypeptide that binds to the binding agent; and (c) comparing the level of polypeptide with a predetermined standard or cut-off value.

In particular examples, the binding agent is an antibody. Antibodies specifically reactive with one or more ps20 polypeptides, or derivatives, such as enzyme conjugates or labeled derivatives, may be used to detect one or more ps20 polypeptides in various samples (e.g. biological materials). They may be used as diagnostic or prognostic reagents and they may be used to detect abnormalities in the levels of one or more ps20 polypeptides, and/or temporal, tissue, cellular, or subcellular location of one or more ps20 polypeptides. Antibodies may also be used to screen potentially therapeutic compounds *in vitro* to determine their effects on viral diseases involving one or more ps20 polypeptides and other conditions. *In vitro* immunoassays may also be used to assess or monitor the efficacy of particular therapies.

Disclosed is also a method for monitoring or diagnosing a viral disease in a subject by quantitating one or more ps20 polypeptides in a biological sample from the subject comprising reacting the sample with antibodies specific for one or more ps20 polypeptides which are directly or indirectly labeled with detectable substances and detecting the detectable substances. In a particular example , ps20 polypeptides are quantitated or measured.

Disclosed is a method for diagnosing or detecting a viral disease is provided comprising:
(a) obtaining a sample suspected of containing one or more ps20 polypeptides;
(b) contacting said sample with antibodies that specifically bind to the ps20 polypeptides under conditions effective to bind the antibodies and form complexes;
(c) measuring the amount of ps20 polypeptides present in the sample by quantitating the amount of the complexes; and
(d) comparing the amount of ps20 polypeptides present in the samples with the amount of ps20 polypeptides in a control, wherein a change or significant difference in the amount of ps20 polypeptides in the sample compared with the amount in the control is diagnostic or indicative of a viral disease.

Disclosed is a method for monitoring the progression of a viral disease in an individual, comprising:
(a) contacting antibodies which bind to one or more ps20 polypeptides with a sample from the individual so as to form complexes comprising the antibodies and one or more ps20 polypeptides in the sample;
(b) determining or detecting the presence or amount of complex formation in the sample;
(c) repeating steps (a) and (b) at a point later in time; and
(d) comparing the result of step (b) with the result of step (c), wherein a difference in the amount of complex formation is indicative of disease, disease stage, and/or progression of the disease in said individual.

The amount of complexes may also be compared to a value representative of the amount of the complexes from an individual not afflicted with a viral disease at different stages. A significant difference in complex formation may be indicative of advanced disease or an unfavourable prognosis.

In examples, a ps20 polypeptide of SEQ ID NOs. 2 or 3 is detected in samples and higher levels, in particular significantly higher levels compared to a control (normal) is diagnostic or indicative of a viral disease.

Antibodies may be used in any known immunoassays that rely on the binding interaction between antigenic determinants of one or more ps20 polypeptides and the antibodies. Immunoassay procedures for *in vitro* detection of antigens in fluid samples are also well known in the art. [See for example, Paterson et al., Int. J. Can. 37:659 (1986) and Burchell et al., Int. J. Can. 34:763 (1984) for a general description of immunoassay procedures]. Qualitative and/or quantitative determinations of one or more ps20 polypeptide in a sample may be accomplished by competitive or non-competitive immunoassay procedures in either a direct or indirect format. Detection of one or more ps20 polypeptides using antibodies can be done utilizing immunoassays which are run in either the forward, reverse or simultaneous modes. Examples of immunoassays are radioimmunoassays (RIA), enzyme immunoassays (e.g. ELISA), immunofluorescence, immunoprecipitation, latex agglutination, hemagglutination, histochemical tests, and sandwich (immunometric) assays. These terms are well understood by those skilled in the art. A person skilled in the art will know, or can readily discern, other immunoassay formats without undue experimentation.

According to an example, an immunoassay for detecting one or more ps20 polypeptides in a biological sample comprises contacting binding agents that specifically bind to ps20 polypeptides in the sample under conditions that allow the formation of first complexes comprising a binding agent and ps20 polypeptides and determining the presence or amount of the complexes as a measure of the amount of ps20 polypeptides contained in the sample. In a particular example, the binding agents are labeled differently or are capable of binding to different labels.

Antibodies may be used to detect and quantify one or more ps20 polypeptides in a sample in order to diagnose a viral disease. Immunohistochemical methods for the detection of antigens in tissue samples are well known in the art. For example, immunohistochemical methods are described in Taylor, Arch. Pathol. Lab. Med. 102:112 (1978). Briefly, in the context of the present disclosure a sample obtained from a subject suspected of having a viral disease is contacted with antibodies, preferably monoclonal antibodies recognizing one or more ps20 polypeptides. The binding of antibodies to ps20 polypeptides is determined by selective staining of the sample by standard immunohistochemical procedures. The same procedure may be repeated on the same sample using other antibodies that recognize one or more ps20 polypeptides. Alternatively, a sample may be contacted with antibodies against one or more ps20 polypeptides simultaneously, provided that the antibodies are labeled differently or are able to bind to a different label.

An antibody microarray in which binding sites comprise immobilized, preferably monoclonal, antibodies specific to a substantial fraction of ps20 polypeptides of interest can be utilized in the present invention. Antibody arrays can be prepared using methods known in the art [(see for example, Zhu et al., Science 293:2101 (2001) and reference 20].

Binding agents (e.g., antibodies) specific for one or more ps20 polypeptides may be labelled with a detectable substance and detected in samples based upon the presence of the detectable substance. Examples of detectable substances include, but are not limited to, the following: radioisotopes (e.g., ³H, ¹⁴C, ³⁵S, ¹²⁵I, ¹³¹I), fluorescent labels (e.g., FITC, rhodamine, lanthanide phosphors), luminescent labels such as luminol; enzymatic labels (e.g., horseradish peroxidase, beta-galactosidase, luciferase, alkaline phosphatase, acetylcholinesterase); biotinyl groups, and predetermined polypeptide epitopes recognized by a secondary reporter. In some embodiments, labels are attached via spacer arms of various lengths to reduce potential steric hindrance. Antibodies may also be coupled to electron dense substances, such as ferritin or colloidal gold, which are readily visualised by electron microscopy.

One of the ways an antibody can be detectably labeled is to link it directly to an enzyme. The enzyme when later exposed to its substrate will produce a product that can be detected. Examples of detectable substances that are enzymes are horseradish peroxidase, beta-galactosidase, luciferase, alkaline phosphatase, acetylcholinesterase, malate dehydrogenase, ribonuclease, urease, catalase, glucose-6-phosphate, staphylococcal nuclease, delta-5-steriod isomerase, yeast alcohol dehydrogenase, alpha-glycerophosphate, triose phosphate isomerase, asparaginase, glucose oxidase, and acetylcholine esterase.

For increased sensitivity in an immunoassay system a fluorescence-emitting metal atom such as Eu (europium) and other lanthanides can be used. These can be attached to the desired molecule by means of metal-chelating groups such as DTPA or EDTA.

A bioluminescent compound may also be used as a detectable substance. Bioluminescence is a type of chemiluminescence found in biological systems where a catalytic protein increases the efficiency of the chemiluminescent reaction. The presence of a bioluminescent molecule is determined by detecting the presence of luminescence. Examples of bioluminescent detectable substances are luciferin, luciferase and aequorin.

Indirect methods may also be employed in which the primary antigen-antibody reaction is amplified by the introduction of a second antibody, having specificity for the antibody reactive against one or more ps20 polypeptides. By way of example, if the antibody having specificity against one or more ps20 polypeptides is a rabbit IgG antibody, the second antibody may be goat anti-rabbit gamma-globulin labelled with a detectable substance as described herein.

Methods for conjugating or labelling the binding agents (e.g. antibodies) discussed above may be readily accomplished by one of ordinary skill in the art. (See for example Inman, Methods In Enzymology, Vol. 34, Affinity Techniques, Enzyme Purification: Part B, Jakoby and Wichek (eds.), Academic Press, New York, p. 30, 1974; and Wilchek and Bayer, "The Avidin-Biotin Complex in Bioanalytical Applications,"Anal. Biochem. 171:1-32, 1988 re methods for conjugating or labelling the antibodies with enzyme or ligand binding partner).

Cytochemical techniques known in the art for localizing antigens using light and electron microscopy may be used to detect one or more ps20 polypeptides. Generally, antibodies may be labeled with detectable substances and one or more ps20 polypeptides may be localised in tissues and cells based upon the presence of the detectable substances.

In the context of the methods disclosed herein, the sample, binding agents (e.g. antibodies specific for one or more ps20 polypeptides), or one or more ps20 polypeptides may be immobilized on a carrier or support. Examples of suitable carriers or supports are agarose, cellulose, nitrocellulose, dextran, Sephadex, Sepharose, liposomes, carboxymethyl cellulose, polyacrylamides, polystyrene, gabbros, filter paper, magnetite, ion-exchange resin, plastic film, plastic tube, glass, polyamine-methyl vinylether-maleic acid copolymer, amino acid copolymer, ethylene-maleic acid copolymer, nylon, silk, etc. The support material may have any possible configuration including spherical (e.g. bead), cylindrical (e.g. inside surface of a test tube or well, or the external surface of a rod), or flat (e.g. sheet, test strip). Thus, the carrier may be in the shape of, for example, a tube, test plate, well, beads, disc, sphere, etc. The immobilized binding agent (e.g. antibody) may be prepared by reacting the material with a suitable insoluble carrier using known chemical or physical methods, for example, cyanogen bromide coupling. An antibody may be indirectly immobilized using a second antibody specific for the antibody. For example, mouse antibody specific for a ps20 polypeptide may be immobilized using sheep anti-mouse IgG Fc fragment specific antibody coated on the carrier or support.

Where a radioactive label is used as a detectable substance, one or more ps20 polypeptides may be localized by radioautography. The results of radioautography may be quantitated by determining the density of particles in the radioautographs by various optical methods, or by counting the grains.

Time-resolved fluorometry may be used to detect a signal. For example, the method described in Christopoulos TK and Diamandis EP Anal Chem 1992:64:342-346 may be used with a conventional time-resolved fluorometer.

One or more ps20 polypeptides antibodies may also be indirectly labelled with an enzyme. For example, the antibodies may be conjugated to one partner of a ligand binding pair, and the enzyme may be coupled to the other partner of the ligand binding pair. Representative examples include avidin-biotin, and riboflavin-riboflavin binding protein. In an example, the antibodies are biotinylated and the enzyme is coupled to streptavidin. In another example, an antibody specific for a ps20 polypeptide antibody is labeled with an enzyme.

In accordance with an example disclosed are means for determining one or more ps20 polypeptides in a sample by measuring one or more ps20 polypeptides by immunoassay. It will be evident to a skilled artisan that a variety of immunoassay methods can be used to measure one or more ps20 polypeptides. In general, an immunoassay method may be competitive or noncompetitive. Competitive methods typically employ an immobilized or immobilizable antibody to one or more ps20 polypeptides and a labeled form of one or more ps20 polypeptides. Sample ps20 polypeptides and labeled ps20 polypeptides compete for binding to antibodies to ps20 polypeptides. After separation of the resulting labeled ps20 polypeptides that have become bound to antibodies (bound fraction) from that which has remained unbound (unbound fraction), the amount of the label in either bound or unbound fraction is measured and may be correlated with the amount of ps20 polypeptides in the test sample in any conventional manner, e.g., by comparison to a standard curve.

In an aspect, a non-competitive method is used for the determination of one or more ps20 polypeptides, with the most common method being the "sandwich" method. In this assay, two antibodies to ps20 polypeptides are employed. One of the antibodies to ps20 polypeptides is directly or indirectly labeled (sometimes referred to as the "detection antibody") and the other is immobilized or immobilizable (sometimes referred to as the "capture antibody"). The capture and detection antibodies can be contacted simultaneously or sequentially with the test sample. Sequential methods can be accomplished by incubating the capture antibody with the sample, and adding the detection antibody at a predetermined time thereafter (sometimes referred to as the "forward" method); or the detection antibody can be incubated with the sample first and then the capture antibody added (sometimes referred to as the "reverse" method). After the necessary incubation(s) have occurred, to complete the assay, the capture antibody is separated from the liquid test mixture, and the label is measured in at least a portion of the separated capture antibody phase or the remainder of the liquid test mixture. Generally it is measured in the capture antibody phase since it comprises ps20 polypeptides bound by ("sandwiched" between) the capture and detection antibodies. In an embodiment, the label may be measured without separating the capture antibodies and liquid test mixture.

In a typical two-site immunometric assay for ps20 polypeptides, one or both of the capture and detection antibodies are polyclonal antibodies or one or both of the capture and detection antibodies are monoclonal antibodies (i.e. polyclonal/polyclonal, monoclonal/monoclonal, or monoclonal/polyclonal). The label used in the detection antibody can be selected from any of those known conventionally in the art. The label may be an enzyme or a chemiluminescent moiety, but it can also be a radioactive isotope, a fluorophor, a detectable ligand (e.g., detectable by a secondary binding by a labeled binding partner for the ligand), and the like. In a particular aspect, the antibody is labelled with an enzyme which is detected by adding a substrate that is selected so that a reaction product of the enzyme and substrate forms fluorescent complexes. The capture antibody may be selected so that it provides a means for being separated from the remainder of the test mixture. Accordingly, the capture antibody can be introduced to the assay in an already immobilized or insoluble form, or can be in an immobilizable form, that is, a form which enables immobilization to be accomplished subsequent to introduction of the capture antibody to the assay. An immobilized capture antibody may comprise an antibody covalently or noncovalently attached to a solid phase such as a magnetic particle, a latex particle, a microtiter plate well, a bead, a cuvette, or other reaction vessel. An example of an immobilizable capture antibody is antibody which has been chemically modified with a ligand moiety, e.g., a hapten, biotin, or the like, and which can be subsequently immobilized by contact with an immobilized form of a binding partner for the ligand, e.g., an antibody, avidin, or the like. In an embodiment, the capture antibody may be immobilized using a species specific antibody for the capture antibody that is bound to the solid phase.

The above-described immunoassay methods and formats are intended to be exemplary and are not limiting.

### Computer Systems

Analytic methods contemplated herein can be implemented by use of computer systems and methods described below and known in the art. Thus, disclosed are computer readable media comprising one or more ps20 markers, and optionally other markers. "Computer readable media" refers to any medium that can be read and accessed directly by a computer, including but not limited to magnetic storage media, such as floppy discs, hard disc storage medium, and magnetic tape; optical storage media such as CD-ROM; electrical storage media such as RAM and ROM; and hybrids of these categories such as magnetic/optical storage media. Thus, the invention contemplates computer readable medium having recorded thereon markers identified for patients and controls.

"Recorded" refers to a process for storing information on computer readable medium. The skilled artisan can readily adopt any of the presently known methods for recording information on computer readable medium to generate manufactures comprising information on one or more ps20 markers, and optionally other markers.

A variety of data processor programs and formats can be used to store information on one or more ps20 markers and other markers on computer readable medium. For example, the information can be represented in a word processing text file, formatted in commercially-available software such as WordPerfect and MicroSoft Word, or represented in the form of an ASCII file, stored in a database application, such as DB2, Sybase, Oracle, or the like. Any number of data processor structuring formats (e.g., text file or database) may be adapted in order to obtain computer readable medium having recorded thereon the marker information.

By providing the marker information in computer readable form, one can routinely access the information for a variety of purposes. For example, one skilled in the art can use the information in computer readable form to compare marker information obtained during or following therapy with the information stored within the data storage means.

Disclosed is a medium for holding instructions for performing a method for determining whether a patient has a viral disease, comprising determining the presence or absence of one or more ps20 markers, and optionally other markers, and based on the presence or absence of the one or more ps20 markers and optionally other markers, determining a viral disease, and optionally recommending a procedure or treatment.

A method is disclosed for diagnosing or detecting a viral disease using a computer having a processor, memory, display, and input/output devices, the method comprising the steps of:
(a) creating records of one or more ps20 markers, and optionally other markers of a viral disease in a sample suspected of containing ps20 markers;
(b) providing a database comprising records of data comprising one or more ps20 markers, and optionally other markers; and
(c) using a code mechanism for applying queries based upon a desired selection criteria to the data file in the database to produce reports of records of step (a) which provide a match of the desired selection criteria of the database of step (b) the presence of a match being a positive indication that the markers of step (a) have been isolated from a sample of an individual with a viral disease.

In an aspect of the invention, the computer systems, components, and methods described herein are used to monitor a viral disease or determine the stage of a viral disease.

### Kits

Disclosed herein are kits for carrying out the methods of the invention. Kits may typically comprise two or more components required for performing a diagnostic assay. Components include but are not limited to compounds, reagents, containers, and/or equipment. A kit may contain microtiter plate wells, standards, assay diluent, wash buffer, adhesive plate covers, and/or instructions for carrying out a method of the invention using the kit. The kit may be a package which houses a container which contains a diagnostic composition and also houses instructions for carrying out a method of the invention to diagnose a viral disease.

The methods described herein may be performed by utilizing pre-packaged diagnostic kits comprising one or more specific ps20 polynucleotides or antibody described herein, which may be conveniently used, e.g., in clinical settings to screen and diagnose patients and to screen and identify those individuals exhibiting a predisposition to developing a viral disease.

Disclosed is a kit for assessing the presence of ps20 polypeptides, wherein the kit comprises binding agents (e.g. antibodies) specific for one or more ps20 polypeptides, or primers or probes for polynucleotides encoding same, and optionally probes, primers or antibodies specific for other markers associated with a viral disease.

A kit may include a container comprising a binding agent as described herein. In an aspect of the invention, the kit includes antibodies or fragments of antibodies which bind specifically to an epitope of one or more ps20 polypeptide and means for detecting binding of the antibodies to their epitopes, either as concentrates (including lyophilized compositions), which may be further diluted prior to use or at the concentration of use. In another aspect, the kit may contain antibodies or antibody fragments which bind specifically to epitopes of one or more ps20 polypeptides and optionally other markers, antibodies against the antibodies labelled with an enzyme; and a substrate for the enzyme.

A kit may be designed to detect the level of ps20 polynucleotides in a sample. Such kits generally comprise at least one oligonucleotide probe or primer, as described herein, that hybridizes to a polynucleotide encoding one or more ps20 polypeptide. Such oligonucleotide may be used, for example, within a PCR or hybridization procedure. Additional components that may be present within the kits include a second oligonucleotide and/or a diagnostic reagent or container to facilitate detection of a polynucleotide encoding one or more ps20 polypeptides.

Disclosed is thus a kit for aiding the diagnosis of a human immunodeficiency virus infection in a subject or for assessing susceptibility of a subject to human immunodeficiency virus infection, said kit comprising a reagent for the specific detection of ps20 expression. Suitably said reagent comprises anti-ps20 antibody. Suitably said antibody comprises IG7 antibody. Said kit may also comprise secondary reagents for detection such as anti-mouse antibodies linked to one or more moieties for visualization such as horseradish peroxidase, alkaline phosphatase, or fluorescent reagent(s).

Disclosed is a kit containing a microarray described herein ready for hybridization to target ps20 polynucleotides, plus software for the analysis of the results. The software to be included with the kit comprises data analysis methods, in particular mathematical routines for marker discovery, including the calculation of correlation coefficients between clinical categories and marker expression. The software may also include mathematical routines for calculating the correlation between sample marker expression and control marker expression, using array-generated fluorescence data, to determine the clinical classification of the sample.

The disclosure relates also to the following:
1. A method for diagnosing a viral disease in a subject comprising:
   (a) obtaining from the subject a biological sample suspected of containing ps20 polypeptides or ps20 polynucleotides;
   (b) assaying for one or more ps20 polypeptides or ps20 polynucleotides in the sample; and
   (c) comparing the amount of ps20 polypeptides or ps20 polynucleotides assayed to a predetermined standard, where detection of amounts of ps20 polypeptides or ps20 polynucleotides that differ significantly from the standard indicates a viral disease or susceptibility or predisposition to a viral disease.
2. A method as described in numbered paragraph 1 wherein the amount of ps20 polypeptides or ps20 polynucleotides assayed is greater than that of a standard and is indicative of a viral disease.
3. A method as described in numbered paragraph 1 or 2 comprising:
   (a) contacting the biological sample with one or more binding agent that specifically binds to the ps20 polypeptides; and
   (b) detecting in the sample amounts of ps20 polypeptides that bind to the binding agents, relative to a predetermined standard or cut-off value, and therefrom determining the presence or absence of the viral disease in the subject.
4. A method as described in numbered paragraph 3 wherein the binding agent is an antibody.
5. A method as described in numbered paragraph 1 or 2 comprising detecting one or more ps20 polynucleotides the sample and relating the detected amount to the presence of the viral disease.
6. A method as described in numbered paragraph 5 wherein the polynucleotide detected is mRNA.
7. A method as described in numbered paragraph 6 wherein the mRNA is detected using an amplification reaction.
8. A method as described in numbered paragraph 7 wherein the amplification reaction is a polymerase chain reaction employing oligonucleotide primers that hybridize to the polynucleotides, or complements of such polynucleotides.
9. A method as described in numbered paragraph 6 wherein the mRNA is .detected using a hybridization technique employing oligonucleotide probes that hybridize to the polynucleotides or complements of such polynucleotides.
10. A method as described in numbered paragraph 9 wherein the mRNA is detected by
   (a) isolating mRNA from the sample and combining the mRNA with reagents to convert it to cDNA; (b) treating the converted cDNA with amplification reaction reagents and primers that hybridize to the polynucleotides, to produce amplification products; (d) analyzing the amplification products to detect an amount of mRNA encoding one or more ps20 markers; and (e) comparing the amount of mRNA to an amount detected against a panel of expected values for normal samples derived using similar primers.
11. A method for diagnosing and monitoring a viral disease in a subject comprising isolating nucleic acids in a sample from the subject; and detecting nucleic acids encoding ps20 polypeptides in the sample wherein the presence of higher levels of nucleic acids encoding ps20 polypeptides in the sample compared to a standard or control is indicative of disease or prognosis.
12. A method for monitoring the progression of a viral disease in a subject, the method comprising: (a) detecting in a sample from the subject at a first time point, one or more ps20 polypeptides or ps20 polynucleotides; (b) repeating step (a) at a subsequent point in time; and (c) comparing levels detected in steps (a) and (b), and thereby monitoring the progression of the viral disease.
13. A method of assessing the efficacy of a therapy for inhibiting a viral infection in a subject, the method comprising comparing: (a) levels of one or more ps20 markers in a first sample obtained from the subject; and (b) levels of the ps20 markers in a second sample obtained from the subject following therapy, wherein a significant difference in the levels of expression of the ps20 markers in the second sample, relative to the first sample, is an indication that the therapy is efficacious for inhibiting the viral infection in the subject.
14. A method as described in any preceding numbered paragraph wherein the sample is obtained from cell lysates or physiological fluids.
15. A method for characterizing CD4 T cells in a subject as permissive for a viral disease comprising:
   (a) obtaining from the subject a sample comprising CD4 T cells;
   (b) detecting or identifying in the sample ps20 polypeptides or ps20 polynucleotides; and
   (c) comparing the detected amount with an amount detected for a standard.
16. A method as described in any preceding numbered paragraph wherein the viral disease is HIV.
17. A method as described in any preceding numbered paragraph wherein the viral disease is influenza.
18. A diagnostic composition comprising an agent that binds to a ps20 polypeptide or hybridizes to a ps20 polynucleotide.

### Further Applications and Advantages

Methods for detecting, diagnosing or monitoring viral diseases in a subject are described comprising measuring ps20 polypeptides or ps20 polynucleotides in a sample from the subject.

The following non-limiting examples are illustrative of the present invention:

### Example 1

This study reports the identification of a novel HIV regulatory protein, ps20 and its diagnostic applications. Human ps20, encoded by the WFDC1 gene [Larsen, M., et al, . J Biol Chem. 1998; 273:4574-4584 and Larsen M, et al Mamm Genome. 2000;11(9):767-73], is a member of the whey-acidic protein (WAP) family, a highly conserved core domain comprising 8-cysteines in a characteristic 4-disulphide bond arrangement identifies WAP proteins. [Wahl SM, et al J Leukoc Biol. 2006]. WAP proteins are mostly secreted factors in mucosal tissue with pleiotropic functions implicated in innate immunity; some are serine protease inhibitors with anti-infective activity [Doumas S, et al Infect Immun. 2005; 73:1271-4 and Hiemstra PS, et al Curr Pharm Des. 2004; 10:2891-905]:

The following materials and methods were used in the study described in this Example.

### Materials and Methods

**Immortalised cell lines and Indicator cells:** were obtained though the AIDS Repository, National Centre for Biological Standards & Controls (NTBSC) (Potters Bar, UK). The indicator T-cell line CEM.G37 was from Dr P Kellam (UCL). Ghost-CCR5 and CEM.G37 indicator cells express green fluorescent protein (GFP) under the control of the HIV-2-LTR and HIV-1-LTR promoter respectively,

**Memory CD4 T-cells:** Peripheral blood mononuclear cells (PBMC) were separated from blood by standard density gradient centrifugation using Lymphopre^{™} (Axis shield, Oslo, Norway). *Ex vivo* CD4 CD45RO+ memory CD4 T-cells were isolated by negative immunomagnetic bead depletion (Dynal Biotech, Oslo, Norway). To obtain activated cells, PBMCs were cultured with 2µg/ml Phytohemagglutinin (PHA) (Biostat Diagnostic Systems, Germany) for 48 hours prior to memory cell isolation. Expanded oligoclonal populations were established from the activated memory fraction by culture for 10-12 days with 30 IU/ml IL2 (Proleukin, Chiron; UK) followed by further rounds of activation conducted every 12-14 days with allogeneic irradiated PBMC, 2µg/ml PHA and 20IU/ml IL2. Cells were fed with fresh 30IU/ml IL2 every 3-4 days and maintained at 5x10⁵ cells/ml in RPMI/Hepes medium /10%FCS/10% human serum (HS) (First Link, UK).

**Clinical samples:** were taken from members of a well-characterized cohort to study the biological and behavioural correlates of non-progression in HIV-1 infection [Easterbrook PJ. Infect. 1999;38(2):71 -3]. PBMC samples from patients who had been recruited for previous immunological studies [Boaz MJ, et al. J Immunol. 2002;169(11):6376-85] were included. Samples from seven treatment naïve, chronically-infected patients with a median time of infection of 14.6 years, median VL 5060 (range <50-437,389) and median CD4 count 804 (range 363-2002) were studied.

**CD4 T-cell cloning and selection of permissive and non-permissive clones:** Clones were generated by the limiting dilution method from CD4 memory fraction isolated from three donors [Vyakarnam, A. et al AIDS 2001;15:1613-26]. Donors included: an HIV-negative blood donor (donor 8); an HIV-infected Long-term Non-Progressor (LTNP 134), and a progressor on ART with a viral load <50 RNA copies/ml plasma (GWS 86). Clones, which exhibited good growth kinetic and confirmed to express CD4+ and CCR5+/CXCR4+, were screened for susceptibility to HIV (X4 strains 2044 & NL4-3;R5 strains BaL &YU2) infection. Clones that restricted replication of either X4 HIV-1 and/or R5 strains (the latter in a β-chemokine-independent manner) were selected for further study alongside permissive clones from the same individual.

**Ps20 identified as candidate HIV regulatory protein by differential Affymetrix screening:** RNA isolation/characterization, Affymetrix (HG-U133A and HG-U133B) array probe synthesis, hybridisation, washing and MAS5 analysis were performed by the Human Genome Mapping Project Centre, Cambridge, UK. Data analysis was performed in collaboration with Sarah Webb and Peter Underhill of the Mammalian Genetics Unit, MRC Harwell. Differential screen a permissive / non-permissive clone pair was conducted under three conditions: at baseline (resting before activation), 6 days after mock infection (and PHA/IL2 activation) and 6 days after HIV-1 infection (and PHA/IL2 activation); productive infection was confirmed by accumulation of HIV-1 Gag p24 in culture supernatant. The large number of transcripts investigated necessitated a tiered approach to analysis. The data was initially filtered to remove genes that had an absent call on both arrays (filter 1). An arbitrary cut-off of 100 was then applied to remove genes whose signal intensity did not exceed 100 in at least one condition (filter 2) based on the observation that data below 100 was associated with high co-efficient of variance. Only those candidate genes that were at least 3-fold and above differentially expressed (filter 3) between the clone pair were considered as the top differentials. A total of 79 differentially expressed candidates were identified. Ps20 was one of three candidates that was differentially expressed in all conditions tested.

**Virus Stocks:** Primary X4 HIV-1 strain 2044 and R5 strain mBaL was propagated in PHA activated PBMC [Vyakamam, A., et al,. AIDS 2001;15:1613-26]. Supernatant from parallel uninfected cultures served for mock infection. Full-length HIV infectious molecular clones were produced by standard transient transfection of 293T cells with purified proviral DNA encoding the HIV-1 molecular clone YU2, or NL4-3. Viral stocks were standardised by HIV-1 Gag-p24 concentrations measured by ELISA (NCI Frederick, HIV-1 p24 Antigen Capture Assay Kit).

**Single cycle infection with replication competent HIV:** 2x10⁵ cells/well were seeded in a 24 well plate, and exposed to modulators overnight prior infection with NL4-3 (10ng po24-CA/million cells). 24 hrs post infection cells were washed x3 with cold PBS, trypsinized (Sigma-Aldrich) for 5min at 37°C, and washed again x3 in PBS with 5% FCS. Cell pellets were resuspended in cold lysis buffer (PBS with 1% Triton X, and 1% NP-40 and stored at -80°C prior to use in p24-CA ELISA assay.

**Spreading HIV infection:** CD4 T-cell clones were activated with irradiated allogeneic PBMC (1:1 ratio) + 2µg/ml PHA + 20 IU/ml IL2. Five days later cells were washed and a total of 1-5 million cells challenged with HIV virus stocks (1-50ng HIV p24-CA/ million cells) in a final volume of 200-300µL. 18 hours later, cells were washed and fresh medium added to a final volume of 1 ml and maintained in 30 IU/ml IL2. HIV-1 p24-CA antigen concentration in cell-free supernatant was measured over time. Experiments were optimised to achieve reproducible virus spread; the X4 HIV-1 strain 2044 [see Vyakarnam, A., et al, AIDS 2001;15:1613-26see 3] was consistently more efficient than X4 strain NL4-3 in spreading infection of primary CD4s/clones, even when both virus stocks were produced in the same producer cell (PHA-stimulated PBMC); on the other hand, immortalised CD4 lines were equally susceptible to both strains irrespective of producer cell (DFL King; PhD thesis, University of London, 2005). Therefore X4 strain 2044 was generally used for spreading infection of primary CD4s.

**Non-quantitative Reverse transcription (RT) PCR of ps20 mRNA:** RNA was isolated with a one-step RT-PCR Kit (QIAGEN, West Sussex, UK). Q solution included in the kit was necessary to remove secondary structure in the GC-rich target sequence. Amplification of full-length ps20 mRNA was optimized for use with 0.2ug template/0.6uM of each primer (MWG Biotech, London, UK) in a total reaction volume of 25ul. Primers were FWD: 5' GCATGCCTTTAACCGGCGTGG 3' , *[SEQ ID NO. 4]*, REV: 5' GCTTACTGAAAGTGCTTCTG 3' *[SEQ ID NO. 5]*. HPRT was used as an internal control with primers FWD: 5' ACCAGTCAACAGGGGGACAT 3' *[SEQ ID NO. 6]*, REV: 5' CGACCTTGACCATCTTTGGA 3' *[SEQ ID NO. 7]*, used at 0.6uM per reaction. All reactions were performed in an Eppendorf™ gradient thermocycler: RT 50°C for 30mins, HotStarTaq initiation 95°C for 15mins, Denaturation 94°C for 30secs, Annealing 56°C 30secs, Extension 72°C for 1min, 35 Cycles, Final extension 72°C 10 mins. Products were visualized by agarose (SIGMA) (1.6-2%) gel electrophoresis containing 0.5mg/ml EtBr for 90 mins at 90V and imaged under UV light.

**Quantitative Real Time PCR for ps20 mRNA:** Total RNA from pre-determined numbers of cells extracted with TRIZOL (Invitrogen) was converted to cDNA (Ambion). Ps20 mRNA was measured with respect to HPRT, using the Quantitect SYBR Green PCR kit (Qiagen, West Sussex, UK), on an ABI Prism 7000 (Applied Biosystems, CA, USA). Cycle parameters were: 2 minutes at 50°C, 15 minutes at 95°C, then 40 cycles (20 seconds at 95°C, 30 secs at 60°C, 30 secs at 72°C) followed by standard dissociation stage. HPRT primer sequence: 5'-ACC AGT CAA CAG GGG GACAT-3' (forward) *[SEQ ID NO. 8]*, 5'-CGA CCT TGA CCA TCT TTG GA-3' (reverse) *[SEQ ID NO. 9]*. Codon optimized ps20 primers were from Qiagen Ltd. For absolute quantitation, an in-vitro transcribed standard curve was generated. The H1-1pBK-CMV plasmid was linearized and used in a T7-transcription MEGAscript(R) kit (Ambion). A log dilution standard of known ps20 mRNA concentrations (1000-0.001pg) spiked with a uniform 1ng Herring sperm cDNA (Promega, Madison, WI, USA) was included in each run along with test samples, positive, negative and RT-controls. A standard curve of ps20 mRNA vs. PCR cycle number (ct) was generated and ps20 molecules per cell calculated from the known MW of ps20.

**Statistical analysis:** Statistical analysis was done in GraphPad PRISM software {PRISM 4 for Macintosh). Group differences were determined by non-parametric test and p<0.05 considered significant

### Results.

### ps20 RNA levels correlate with increased HIV-1 spread and HIV infection

Having identified ps20 by screening a HIV. permissive (P)/ resistant clone pair, its expression was first examined in an expanded panel of six clones from three donors. An association was found between ps20 mRNA level and increased HIV spread in diverse CD4 T-cell populations (clones, primary CD4 T-cells and immortalized lines -H9/HUT) of the same genetic origin in each case (Figure 1).

Ps20 mRNA levels were shown to be higher in HIV+ subjects compared to control subjects (Figure 2).

### Cellular profile of a ps20+ HIV permissive CD4 T cell shows at an early/intermediate stage of differentiation

The well-characterised primary P/NP clones (see above) were used to build a cellular profile of a ps20+ permissive CD4 T-cell. Data summarised in Table 1 provide the following picture: (i) Cellular resistance is not associated with lower HIV. receptor / coreceptor expression. Indeed, the three ps20^{hi} permissive clones had up-to 2-fold lower expression of the CCR5 coreceptor. (ii) All clones were memory cells at an early/intermediate differentiation stage judged by being CD45RO+/CD25+/CD28+/CD57- (iii) There were no apparent differences in the proliferative capacity of P/NP cells or their Thl/2 cytokine profile. Both P and NP clones could be Th2 or Th0 (Th1 clones were not examined as previous studies had highlighted Th1 cells to be less permissive to HN than Th2 and Th0 cells [Vyakamam, A., et al, 1995. Immunology 86:85-96].

### Discussion

This study illustrates that differential expression of a novel secreted factor, ps20, can contribute to hierarchies in susceptibility to HIV. infection with the most permissive memory cells being ps20+. ps20 marks a subset of memory CD4s at an early differentiation stage that have previously been described to be preferentially targeted by HIV *in vivo* (CD45RO+/CD28+/CD57-) whose preservation is likely associated with non-progression [Harari A, et al, Immunol Rev. 2006 Jun;2] 1:236-54; Brenchley, J. M, et al, Journal of Virology 2004;78:1160-1168; Brenchley JM, et al., J Virol. 2006 Ju1;80(14):6801-9; and Boar MJ, et al, J Immunol. 2002;169(11):6376-85]. The observation that memory cells from HIV patients constitutively express ps20 mRNA therefore suggests that these cells are not in a resting state, consistent with *in vivo* activation [Stevenson M. NatMed. 2003;9:853-60 and Harari A, Immunol Rev. 2006 Jun;2] 1:236-54]. These observations suggest ps20+ cells may be preferentially targeted *in vivo* and promote virus spread by rendering cells more susceptible to infection; thereby playing a role in disease pathogenesis.

ps20 can enhance HIV infection. HIV exploitation of WAPs, like ps20, may reflect virus adaptation to their anti-infective functions and/or exploitation of the physiological properties of this class of proteins. ps20 may function as a secreted extracellular matrix protein and that both the cell adhesion and chemokine-like functions of ps20 could be co-opted by HIV as permissive components of infection pathways.

### Example 2: Assays to measure ps20

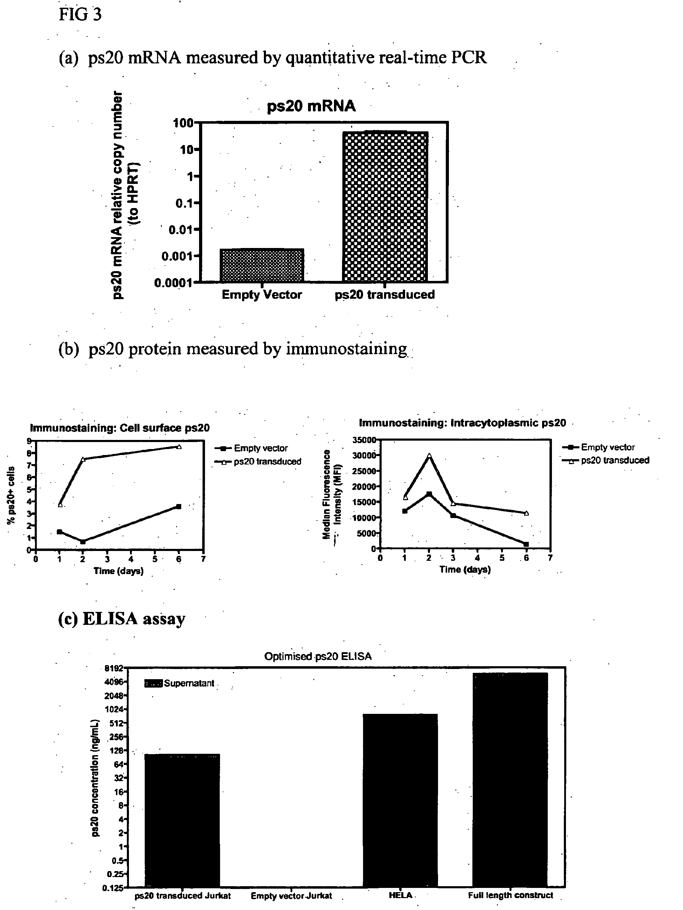

Fig 3(a) shows ps20 mRNA measured by quantitative real-time PCR (qRT-PCR).

### Cells and constructs used to optimize assay:

Stable expression of ps20 in Jurkats by retroviral transduction: The ps20 sequence was cloned into the EcoRI site of pCxCR (Empty Vector, EV), an MMLV-based bi-cistronic retroviral vector, which expresses Red Fluorescent Protein (RFP) under the control of a CMV promoter (kind gift Dr G Towers, University College London) and named pCpsCR. To generate retroviral particles, 293T cells were transiently transfected with either pCxCR or pCpsCR, along with the packaging construct pCpg (MMLV gag/pol), and an envelope construct encoding VSV-G (pMD.G). 48 hours after transfection, cell free supernatants were harvested. 2x10⁵ CCR5+ Jurkats were cultured 3 times with 50% total volume of retrovirus containing supernatant over a 3 day period. RFP expression was used to sort transduced cells by flow cytometry. Jurkat cells transduced with empty vector and those transduced with ps20 were then used to optimize the following assays for ps20:

### qRT-PCR

Total RNA from pre-determined numbers of cells extracted with TRIZOL (Invitrogen) was converted to cDNA (Ambion). Ps20 mRNA was measured with respect to a common house-keeping gene (beta actin or HPRT), using the Quantitect SYBR Green PCR kit (Qiagen, West Sussex, UK), on an ABI Prism 7000 (Applied Biosystems, CA, USA). Cycle parameters were: 2 minutes at 50°C, 15 minutes at 95°C, then 40 cycles (20 seconds at 9.5°C, 30 secs at 60°C, 30 secs at 72°C) followed by standard dissociation stage. HPRT primer sequence: 5'-ACC AGT CAA CAG GGG GACAT-3' (forward), 5'-CGA CCT TGA CCA TCT TTG GA-3' (reverse). Codon optimized ps20 primers were from Qiagen Ltd. For absolute quantitation, an in-vitro transcribed standard curve was generated. The H1-1pBK-CMV plasmid was linearized and used in a T7-transcription MEGAscript(R) kit (Ambion). A log dilution standard of known ps20 mRNA concentrations (1000-0.001 pg) spiked with a uniform 1 ng Herring sperm cDNA (Promega, Madison, WI, USA) was included in each run along with test samples, positive, negative and RT- controls. A standard curve of ps20 MRNA vs. PCR cycle number (ct) was generated and ps20 molecules per cell calculated from the known MW of ps20.

Data in fig 3(a) shows the mean relative copy number of ps20 mRNA in empty vector control versus ps20 transduced Jurkats in three biological repeat assays and confirms the ps20 transduced population to have higher ps20 mRNA relative to empty vector control.

fig 3(b) shows ps20 protein measured by immunostaining

Anti-ps20 antibodies:
Monoclonal : 10 mg of purified recombinant human ps20 was used as an antigen. Hybridoma preparation and initial antibody screening was performed by Zymed (www.zymed.com). Primary bleeds were screened by ELISA with 96 well plate coated with purified ps20-V5-His protein at 0.1 µg / well with standard protocols. One clone 1G7A9H5 (IG7) gave a high reading, and is particularly useful in the invention.
Rabbit polyclonal 202-254: was generated by a standard protocol developed by Eurogentec Ltd (Belgium using peptide immunisation. A 15-mer peptide covering amino acid 206-220 was predicted by a standard algorithm to be immunogenic and was used by Eurogentec Ltd to generate a polyclonal antibody in rabbits using their proprietary protocol that was affinity purified against the peptide.

Immunostaining: For intracytoplasmic staining, 1 million ps20 transduced and empty vector transduced Jurkat cells were, washed and permeabilised (www.andergrub.com). For surface staining live unfixed cells were stained. Immunostaining involved a standard protocol. 1-2x10⁵ permeabilized cells were compared with cell that were not permeabilised were incubated for 30 min on ice with 1/100-1/500 dilution of anti-ps20 antibody, IG7, washed and then stained with a 1/200 dilution of F(ab)₂ FITC conjugated goat anti-mouse IgG ( Sigma) for 20 minutes on ice. Samples were then washed, fixed in PBS+2%FCS+2%formaldehyde and acquired on a FACSCalibur (BD Biosciences) using Cell Quest software (BD Biosciences).

Fig 3(b) shows that frequency of surface ps20 positive cells and the total median fluorescence intensity following intracytoplasmic (IC) cytokine staining after background subtraction due to secondary antibody/ isotype control. Immunostaining confirms the ps20 transduced population to have higher surface and IC staining for ps20 relative to empty vector control and also shows normal turnover of the ps20 protein these populations.

Fig 3c shows ELISA assay for ps20: Nunc 96-well plates were first coated with 1ug/mL of rabbit polyclonal 202-254 anti-ps20 antibody in PBS over-night at 4°C. A blocking step with PBS containing 1% BSA was carried out for 2H at room temperature then the plate was washed 3 times with 200µL of PBS-0.2% Tween-20 and the samples, either neat or diluted in PBS-1% BSA, were added to the plate for 2H at room temperature. Mouse monoclonal IG7 anti-ps20 antibody conjugated to horse radish peroxydase was added at a final dilution of 1:500 (1.44 ug/ml final concentration) in PBS-1% BSA-0.2% Tween-20 and incubated for 2H at room temperature. Following each incubation step, plates were washed 6 times with 200µL of PBS-0.2% Tween-20. 150µL of substrate OPD (Sigma) were used for colorimetric analysis and the reaction was stopped with 50 µL of 4M sulfuric acid. Optical densities were determined at a wave length of 492 nm in a BioRade ELISA plate reader. Ps20 concentrations were determined in relation to a standard curve using the recombinant ps20 protein of known concentration. Background values obtained with PBS-BSA alone were subtracted in each case.

Fig 3c shows the concentration of ps20 in a number of cell cultures. Ps20 transduced and empty vector Jurkats and HeLa cells (which we previously reported to be high in ps20 mRNA (Vyakarnam A JVI 2008) were cultured at 500,000 cells per ml in RPMI/10% FCS and culture supernatant harvested 72 hours later. In addition culture supernatants harvested by transfecting 293T cells with a construct encoding full-length human ps20 was used a positive control. Ps20 concentration was determined using recombinant ps20 as a standard after subtracting background in control wells containing medium alone. Data confirms higher secreted ps20 in the ps20 transduced versus empty vector transduced Jurkat cells (where levels were below assay detection).

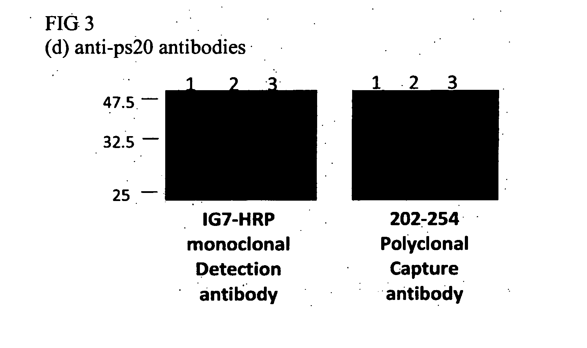

Fig 3(d): Western blot analysis of recombinant ps20: Samples were treated according to the manufacturer's instructions and run on a 12% NuPage Bis-Tris gel (Invitrogen). All membrane wash steps were carried out with Tris-buffered saline plus 0.1 % Tween-20. The membrane blocking steps were done in 5% milk, 2.5% fetal calf serum (FCS) in Tris-buffered saline plus 0.1 % Tween-20, and the membrane was probed with the anti-ps20 Ab IG7 or polyclonal 202-254 at a final dilution of 1:1,000 followed by a horseradish peroxidase-conjugated goat anti-mouse or goat anti-rabbit Ab (Pierce) at a final dilution of 1:1,000. Western blots were developed using an ECL plus Western blotting detection system (Amersham Biosciences, United Kingdom), according to the manufacturer's protocols.

Fig 3(d) confirms that both the IG7 monoclonal and the rabbit 202-254 polyclonal detect a single band of predicted molecular weight for ps20 in the recombinant ps20 preparation.

### Example 3: ps20 MRNA levels correlate with in vitro HIV infection.

I million cells (clones, primary CD4 CD45R0 & H9/HUT 78) were infected overnight with virus dose standardised on HIV-Gag p24-CA concentration (2ng 2044 or 4ng NL4-3 strains). Expanded CD4s and clones were stimulated with allogeneic APC/PHA/IL2 for 5 days prior infection and cultured at 2x10⁵/ml in 30IU/mlIL2 post infection. Mean peak p24-CA levels (day 8) in three biological replicate cultures is shown. qRT-PCR for ps20 per population was determined in triplicate at the time of infection and mean ps20 molecules per cell shown.

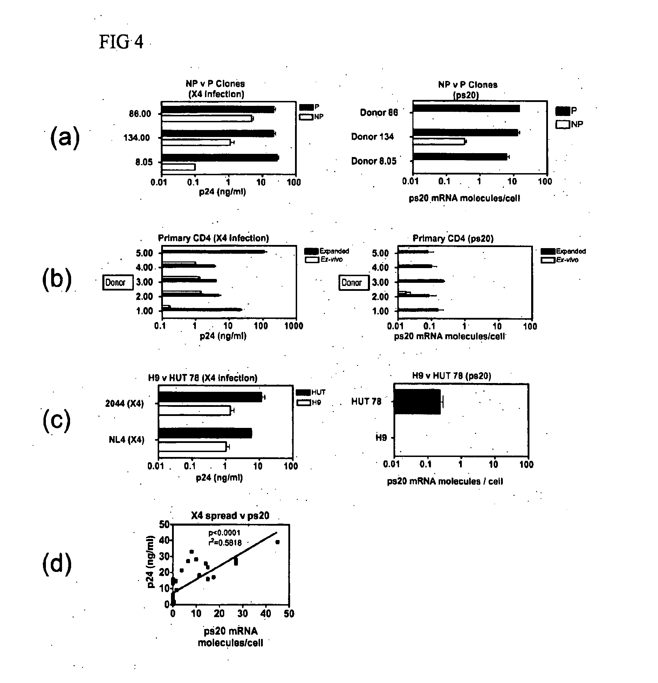

Fig 4(a)Comparison of non-permissive (NP) v permissive (P) counterpart clones from donors 8, 134 and 86 with 2044 (X4) strain. (b) *Ex vivo:* CD4 CD45RO+ T-cells were infected with 2044, then cultured in 30IU/ml IL2. Expanded: Purified CD4+ CD45RO+ T-cells were expanded by two rounds of stimulation with allogeneic PBMC/PHA/IL2 (see M&M), then infected with 2044 and maintained in 30IU/ml IL2 post infection. (c) Comparison of H9 v HUT 78 immortalized cells infected with NL4-3 or 2044 strains. (d) Statistical correlation of peak p24-CA levels v ps20 mRNA molecules per cell for each population was derived on GraphPad PRISM software. Two-tailed P-value derived by non-parametric Spearman's Correlation is shown. Goodness of fit R² derived by linear regression analysis is shown.

Figure 4 confirms a linear relationship between endogenous ps20 mRNA levels and HIV infection.

### Example 4: ps20 mRNA is elevated in HIV infection in vivo.

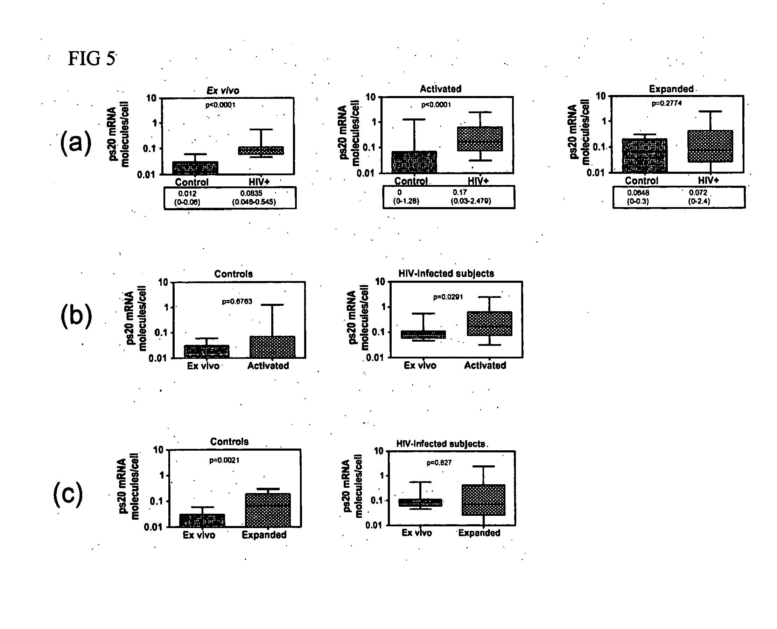

*Fig5(a)* *Ex vivo:* refers to CD4 CD45RO+ T-cells freshly isolated from PBMC by negative selection. Activated: refers to CD4+ CD45RO+ T-cells isolated from 48 hour PHA activated PBMC. Expanded cells refer to CD4+ CD45RO+ T-cells expanded with two rounds of activation with allogeneic PBMC/PHA/IL2. Cells from six control donors and seven HIV+ subjects were examined for ps20 mRNA by qRT-PCR; mean ps20 molecules per cell from 3-7 replicates for each sample is shown. Group differences were determined by non-parametric Mann-Whitney test, median and range shown. (b) Ps20 mRNA level by qRT-PCR as described in (a) above were analysed. Ps20 level in activated v *ex vivo* samples in control and HIV+ subjects are shown. (c) Ps20 mRNA level by qRT-PCR as described in (a) above were analysed. Ps20 level in expanded v *ex vivo* samples in control and HIV+ subjects are shown.

Data in fig 5 show that ps20 RNA is elevated in ex vivo Cd4 CD45RO+ T- cells taken from HIV-infected versus control uninfected subjects.

### Example 5: ps20 protein is elevated in plasma of chronic HIV-infected subjects

Plasma samples from donors confirmed to be negative for a routine HIV. antibody test (N=80) and 10 HIV seropositive donors was collected by centrifuging EDTA blood for 10 minutes at 2500 rpm for room temperature. 100uL of each undiluted plasma was measured for ps20 protein using our standardised ELISA assay and concentrations assessed on a standard curve of known concentrations of recombinant ps20. Group comparison was conducted using Mann-whitney t test.

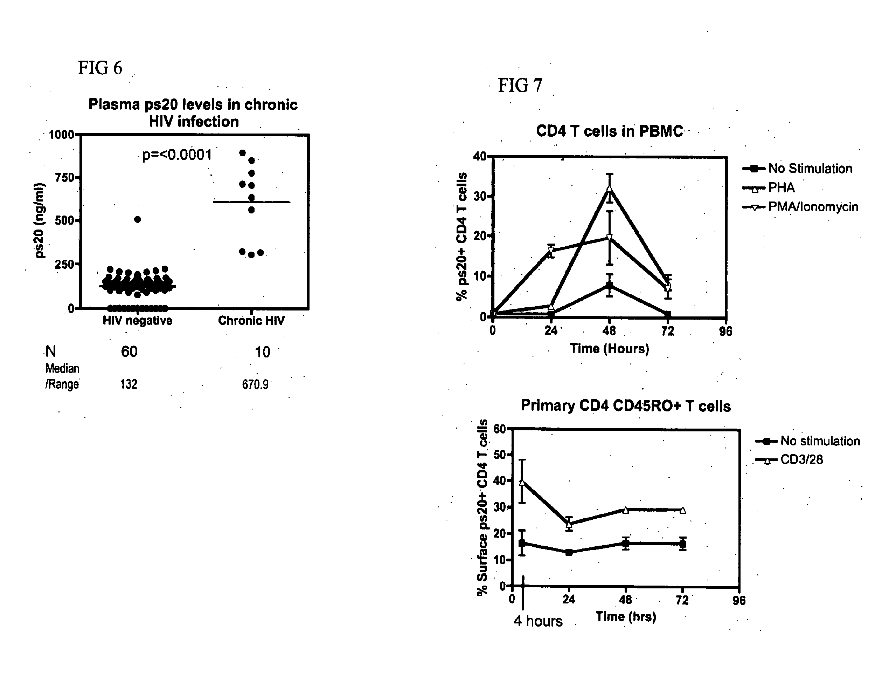

Data in fig 6 shows significantly higher levels of plasma ps20 protein in the HIV seropositive versus seronegative control donors.

### Example 6: In vitro T cell activation up-regulates cell surface ps20.

Human peripheral blood mononuclear cells (PBMC) and CD4+CD45RO+ T cells isolated from PBMC by negative isolation using standard antibody coated magnetic bead technology were cultured at 500,000 cells per well in a final volume of 1ml of RPMI/10% Human serum in a 24-well plate either in the presence of 2ug/ml phytohaemagglutinin (PHA) or with 10ng/ml PMA+1ng/ml Ionomycine. Controls included cells cultured in the absence of these stimuli. Cell surface ps20 was measured by a standard direct immunostaining protocol. Cells were stained with a combination of markers to clearly identify ps20+ T cells: CD3/CD4 and ps20 using the IG7 monolconal directly conjugated to FITC. Samples were then washed, fixed in PBS+2%FCS+2%formaldehyde and acquired on a FACSCalibur (BD Biosciences) using Cell Quest software (BD Biosciences).

### The mean frequency of ps20+ CD3+CD4+ T cells within PBMC and the purified CD4 CD45RO+ T cell fraction from three different donors is shown in a time course assay. Data show that in both populations T cells activation upregulates surface ps20 above the un-stimulated control.

### Example 7: In vitro HIV infection up-regulates ps20

Random oligoclonal CD4 T cell lines were generated by activating purified CD4 T cells from PBMC with a standard protocol activation cocktail consisting of 2ug/ml PHA, irradiated allogeniec PBMC feeders and 30 IU/ml IL2. Cells were fed evry 12-14 days with the activation cocktail and every 3-4 days with IL2 alone.. 5 days after feeding with the activation cocktail, 500, 000 of the oligoclonal cells or Jurkat CD4 T cells were seeded in a final volume of 1 ml 301U/ml IL2 in 24-well plate and either stimulated with 10ng/ml PMA/1ng/ml Ionomycin or with the X4 HIV-1 strain NL4-3 (offering 2ng virus stock per/100,000. cells). Surface ps20 expression was tracked by direct immunostaining with IG7-FITC or isotype control. Samples were then washed, fixed in PBS±2%FCS+2%formaldehyde and acquired on a FACSCalibur (BD Biosciences) using Cell Quest software (BD Biosciences).

The mean frequency of ps20+ oligoclonal T cells from three different donors and from Jurkats is shown in a time course assay. Data show that in both populations T cells activation and HIV infection can independently upregulate surface ps20 above the un-stimulated control.

### Example 8: ps20+ are preferentially susceptible to HIV infection compared to ps20- cells: therefore ps20 could be a marker of those cells that are most vulnerable to HIV infection

CD4+CD45RO+ T cells isolated from PBMC by negative isolation using standard antibody coated magnetic bead technology were cultured at 500,000 cells per well in a final volume of 1ml .of RPMI/10% Human serum in a 24-well plate with anti-CD3/28 expander beads (Dynal) and 30 IU/ml IL2. Similarly Jurkat CD4 T cells were seeded in a final volume of 1 ml RPMI/10% FCS in 24-well plate. Both populations were infected with the HIV. The primary cells were infected with a primary virus isolate 2044 and Jurkat cells with NL4-3. Control uninfected cultures were maintained in parallel. Surface ps20 expression was tracked by direct immunostaining with IG7-FITC or isotype control and correlated with productive HIV infection by staining for intracellular HIV-1 Gag p24 antigen. (Antibody KC57 conjugated to PE from Coulter Ltd). Samples were washed, fixed in PBS+2%FCS+2%formaldehyde and acquired on a FACSCalibur (BD Biosciences) using Cell Quest software. (BD Biosciences) and analysed for 2-colour immunofluorescence.
(a) The mean frequency of p24+ cells within the ps20+ and ps20- fractions is shown in populations from three different donors from three different donors or (b) Jurkat cells. In both cases HIV infection was higher in the ps20+ compared to the ps20- fractions. Background p24 staining in uninfected cultures was subtracted and found to be less than 1%/.

**Table 1. Cellular profile of P/NP clones**

| | ***Clone*** | | | | | |
|---|---|---|---|---|---|---|
| | ***Pair 1 (HIV negative donor)*** | | ***Pair 2 (HIV*+, *LTNP)*** | | ***Pair 3 (HIV*+, *receiving HEART)*** | |
| | ***NP*** | ***P*** | ***NP*** | ***P*** | ***NP*** | ***P*** |
| ***CD3*** | 91.75 | 91.72 | 72 | 84 | 76.67 | 76.8 |
| ***CD4*** | 92.36 | 94.39 | 65 | 75 | 68.42 | 84.95 |
| ***CXCR4*** | 15.69 | 10.4 | 65 | 39 | 42.36 | 21.73 |
| ***CCR5*** | 12.13 | 6.39 | 41.5 | 23 | 24.37 | 16.18 |
| ***CD45R0*** | 98.37 | 98.19 | 93.9 | 74 | 86.89 | 96.49 |
| ***CD25*** | 93.95 | 98.28 | 91.3 | 93.1 | 79.12 | 85.82 |
| ***CD28*** | 32.53 | 46.32 | 38.3 | 17.7 | 71 | 69.98 |
| ***CD57*** | 2.22 | 0.65 | 1.9 | 2.1 | 0.97 | 0.54 |
| ***Fold increase** in **cell number**** | 5.5 | 6 | 5.7 | 7 | 6.7 | 5.7 |
| ***% IFNγ*+** | 0.1 | 0.01 | 0.06 | 0 | 0.99 | 4.31 |
| ***%IL4*+** | 88 | 72 | 89.88 | 88.25 | 11.5 | 8.3 |
| ***% IFNγ*+ *and IL4*+** | 1.3 | 1.5 | 5.97 | 2.33 | 78.46 | 66.27 |

| | | | | | | |
|---|---|---|---|---|---|---|
| * fold increase in cell number 7 days after activation with PHA, allogeneic irradiated PBMC and IL2. | | | | | | |

Numbers denote number of cells expressing a given CD marker as determined by standard flow cytometry using directly conjugated antibodies relative to isotype control. Intracytoplasmic cytokine staining to enumerate frequencies of IFN- and IL4+ cells was as previously described [Ciuffi, A., et al, Infection. 2004 J. Virol. 78:10747-10754]. Two-colour immunofluoresecence was used to determine the frequency of cells expressing IL4 but no IFNγ and vice versa as well as number of cells expressing both cytokines.

### Sequence Listing

SEQ ID NO.: 1
   NM_021197 1396 bp mRNA linear
   Homo sapiens WAP four-disulfide core domain 1 (WFDC1), mRNA 
1 agccaccatc gaggaagggg catgtgctgg acgcggacac atgatccgag ggaccctgct
61 gggtggaact aagaaagtcc agcagactgt gcatgctcct gtccccactc acaggcccac
121 gcagcgaggg gggcccctct tctgtgtgcg tctggaaggt cgctgcccag ggaggaaatg
181 cctttaaccg gcgtggggcc gggcagctgc aggaggcaga tcatccgggc tctgtgcctc
241 ttgctacttc tcctccacgc cggctctgcc aagaatatct ggaaacgggc attgcctgcg
301 aggctggccg agaaatcccg tgccgaggag gcgggcgcgc ccggcggccc ccggcagccc
361 cgagcagacc gctgcccgcc gcctccgcgg acgctgcccc ccggcgcctg ccaggccgcg
421 cgctgtcagg cggactccga gtgcccgcgg caccggcgct gctgctacaa cggatgcgcc
481 tacgcctgcc tagaagctgt gccgcccccg ccagtcttag actggctggt gcagccgaaa
541 cctcgatggc ttggtggcaa tggctggctc ctggatggcc ctgaggaggt gttacaagca
601 gaggcgtgca gcaccacgga ggatggggcc gaacccctgc tctgtccctc gggctatgag
661 tgccacatcc tgagcccagg tgacgtggcc gaaggtatcc ccaaccgtgg gcagtgcgtc
721 aagcagcgcc ggcaagcaga tgggcgaatc ctacgacaca aactttacaa agaatatcca
781 gaaggtgact caaagaatgt ggcagaacct ggaaggggac aacagaagca ctttcagtaa
841 agcaacggca agcagctagg ttgcaagaac attcctctac tttctgctaa gccttggaaa
901 cagttgggaa aagtagtttg accctcacag ttcacattca gctcagcaga gcaagacccc
961 agagatgctt agagacagga cacctggccc tcaaacccag tttggcccag cctggttggg
1021 tgactttgtg ggagccactt aacagctctg ggtccctgtt ttaccatcct gggagcaagg
1081 ccctgcagct ccacgagacc tttaccccgg gaagaagccg ccgcccatga aagcatttct
1141 gaagcccctt tctaagacaa ggctcagcat cttgatattt ttgacagatt cctcccaagt
1201 ctggctctgg gaggtatgta cccatctcaa atgttcccaa gataaattca tccttcagga
1261 aatggaaatg aacttgctta ctaatgtgtg attcctagtt gtagccaccg gatgtgctga
1321 ggcctaaatg ttagcaggtg ggaggaggcc acagaacaat aaaaacaacc aaataagaaa
1381 aaaaaaaaaa aaaaaa
SEQ ID.NO. 2
   NP_067020 220 aa linear
   WAP four-disulfide core domain 1 precursor [Homo sapiens]. 
1 mpltgvgpgs crrqiiralc llllllhags akniwkralp arlaeksrae eagapggprq
61 pradrcpppp rtlppgacqa arcqadsecp rhrrccyngc ayacleavpp ppvldwlvqp
121 kprwlggngw lldgpeevlq aeacsttedg aepllcpsgy echilspgdv aegipnrgqc
181 vkqrrqadgr ilrhklykey pegdsknvae pgrgqqkhfq
SEQ ID NO. 3
   EAW95486 220 aa linear
   WAP four-disulfide core domain 1, isoform Cry_8 [Homo sapiens]
   EAW95487 220 aa linear
   WAP four-disulfide core domain 1, isoform CRA_a [Homo sapiens]
AAG166647 220 aa
   prostate stromal protein ps20 [Homo sapiens].
Q9HC57 220 aa linear
   WAP four-disulfide core domain protein 1 precursor (Prostate stromal protein ps20) (ps20 growth inhibitor). 
1 
61 
121 
181 
SEQ ID NO. 4
   5' GCATGCCTTTAACCGGCGTGG 3'
SEQ ID NO 5
   5' GCTTACTGAAAGTGCTTCTG 3'
SEQ ID NO 6
   5' ACCAGTCAACAGGGGGACAT 3'
SEQ ID NO 7
   5' CGACCTTGACCATCTTTGGA 3'
SEQ ID NO 8
   5'-ACC AGT CAA CAG GGG GACAT-3'
SEQ ID NO 9
   5'-CGA CCT TGA CCA TCT TTG GA-3'

## Claims

1. A method of aiding the diagnosis of a human immunodeficiency virus infection in a subject, said method comprising
(i) providing an *in vitro* sample from the subject
(ii) determining the level of ps20 in said sample
(iii) comparing the level of ps20 of (ii) with the level of ps20 in an uninfected reference sample,
wherein a higher level of ps20 in the sample from the subject compared to the uninfected reference sample indicates an increased likelihood of human immunodeficiency virus infection in said subject.

2. A method according to claim 1, wherein the sample comprises a sample of CD4+ T cells from the subject, and wherein determining the level of ps20 in said sample comprises determining the level of ps20 expression of said T cells.

3. A method according to claim 1, wherein the sample comprises a sample of blood plasma from said subject.

4. A method according to claim 3 wherein the ps20 level is determined by ELISA assay of the blood plasma sample.

5. A method according to claim 1, wherein the sample comprises a sample of nucleic acid from said subject, and wherein determining the level of ps20 in said sample comprises determining the amount of ps20 transcript present in said sample.

6. A method of assessing susceptibility of a subject to human immunodeficiency virus infection, said method comprising
(i) providing an *in vitro* sample from the subject
(ii) determining the level of ps20 in said sample
(iii) comparing the level of ps20 of (ii) with the level of ps20 in a normal reference sample,
wherein a higher level of ps20 in the sample from the subject compared to the normal reference sample indicates an increased susceptibility to human immunodeficiency virus infection in said subject.

7. A method according to claim 6, wherein the *in vitro* sample is an *in vitro* sample of CD4+ T cells from the subject, and wherein determining the level of ps20 comprises determining the level of ps20 expression in said T cells.

8. A method according to any of claims 1 to 4, claim 6 or claim 7 wherein the level of ps20 expression is determined by assaying binding by an anti-ps20 antibody.

9. A method according to claim 9 wherein said anti-ps20 antibody comprises monoclonal IG7 antibody.

10. A method for monitoring the progression of a viral disease in a subject, the method comprising: (a) detecting in an *in vitro* sample from the subject at a first time point, one or more ps20 polypeptides or ps20 polynucleotides; (b) repeating step (a) at a subsequent point in time; and (c) comparing levels detected in steps (a) and (b), and thereby monitoring the progression of the viral disease.

11. A method according to claim 10 wherein step (a) comprises contacting antibodies which bind to one or more ps20 polypeptides with an *in vitro* sample from the subject so as to form complexes comprising the antibodies and one or more ps20 polypeptides in the sample and determining or detecting the presence or amount of complex formation in the sample; and wherein a difference in the amount of complex formation is indicative of disease, disease stage, and/or progression of the disease in said individual.

12. A method of assessing the efficacy of a therapy for inhibiting a viral infection in a subject, the method comprising performing the method of claim 10 wherein the subsequent point in time of step (b) is following therapy, and wherein a significant difference in the levels of expression of the ps20 markers in the second sample, relative to the first sample, is an indication that the therapy is efficacious for inhibiting the viral infection in the subject.

13. A method for determining the severity of a viral disease, in a patient, comprising the steps of (a) contacting an *in vitro* biological sample obtained from a patient with one or more binding agent that specifically binds to one or more ps20 polypeptides; and (b) detecting in the sample an amount of ps20 polypeptides that bind to the binding agent, relative to a predetermined standard or cut-off value, and therefrom determining the severity of a viral disease in the patient.

14. A method according to any of claims 10 to 13 wherein the viral disease is HIV or influenza.

15. A method according to claim 13 wherein the viral disease is HIV.

## Patentansprüche

1. Verfahren zur Unterstützung der Diagnose einer Infektion mit einem menschlichen Immundefekt-Virus in einem Probanden, wobei das Verfahren aufweist
(i) Bereitstellen einer *in vitro* Probe des Probanden
(ii) Bestimmen des Levels von ps20 in der Probe
(iii) Vergleichen des Levels des ps20 von (ii) mit dem Level von ps20 in einer nicht infizierten Referenzprobe, wobei ein höherer Level von ps20 in der Probe des Probanden, verglichen mit der nicht infizierten Referenzprobe, eine gesteigerte Wahrscheinlichkeit einer Infektion mit einem menschlichen Immundefekt-Virus in dem Probanden anzeigt.

2. Verfahren nach Anspruch 1, wobei die Probe eine Probe von CD4+T-Zellen des Probanden aufweist und wobei das Bestimmen des ps20-Levels in der Probe das Bestimmen des Levels der ps20-Expression der T-Zellen in der Probe umfasst.

3. Verfahren nach Anspruch 1, wobei die Probe eine Blutplasmaprobe des Probanden aufweist.

4. Verfahren nach Anspruch 3, wobei das ps20-Level durch ein ELISA-Assay der Blutplasmaprobe bestimmt wird.

5. Verfahren nach Anspruch 1, wobei die Probe eine Nukleinsäure-Probe des Probanden aufweist, und wobei das Bestimmen des ps20-Levels in der Probe das Bestimmen der Menge von ps20-Transkript aufweist, die in der Probe vorliegt.

6. Verfahren zum Abschätzen der Empfänglichkeit eines Probanden für eine Infektion mit einem menschlichen Immundefekt-Virus, wobei das Verfahren aufweist
(i) Bereitstellen einer *in vitro* Probe des Probanden
(ii) Bestimmen des Levels von ps20 in der Probe
(iii) Vergleichen des Levels von ps20 von (ii) mit dem Level von ps20 in einer normalen Referenzprobe, wobei ein höherer Level von ps20 in der Probe des Probanden, verglichen mit der normalen Referenzprobe, eine gesteigerte Empfänglichkeit für eine Infektion mit einem menschlichen Immundefekt-Virus des Probanden anzeigt.

7. Verfahren nach Anspruch 6, wobei die *in vitro* Probe eine *in vitro* Probe von CD4+T-Zellen des Probanden ist, und wobei das Bestimmen des Levels von ps20 das Bestimmen des Levels der ps20-Expression in den T-Zellen umfasst.

8. Verfahren nach irgendeinem der Ansprüche 1 bis 4, Anspruch 6 oder Anspruch 7, wobei der Level der ps20-Expression durch das Untersuchen des Bindens durch einen Anti-ps20-Antikörper aufweist.

9. Verfahren nach Anspruch 9, wobei der Anti-ps20-Antikörper monoklonale IG7 Antikörper aufweist.

10. Verfahren zur Überwachung des Fortschreitens einer viralen Erkrankung in einem Probanden, wobei das Verfahren aufweist: (a) in einer *in vitro* Probe eines Probanden zu einem ersten Zeitpunkt Detektieren eines oder mehrere ps20-Polypeptide oder ps20-Polynukleotide; (b) Wiederholen von Schritt (a) zu einem nachfolgenden Zeitpunkt; und (c) Vergleichen der Level, die in Schritten (a) und (b) detektiert worden sind, und hierdurch Überwachen des Fortschreitens der viralen Erkrankung.

11. Verfahren nach Anspruch 10, wobei Schritt (a) das Kontaktieren von Antikörpern, die an einen oder mehrere ps20-Polypeptide mit einer *in vitro* Probe des Probanden binden, um Komplexe zu bilden, die die Antikörper und eines oder mehrere ps20-Polypeptide in der Probe aufweisen, und Bestimmen oder Detektieren der Gegenwart oder der Menge der Komplexe in der Probe, und wobei ein Unterschied der Menge der Komplexe für die Krankheit, die Erkrankungsphase und/oder das Fortschreiten der Erkrankung in besagtem Individuum indikativ ist.

12. Verfahren zur Überprüfung der Wirksamkeit einer Therapie zur Inhibierung einer viralen Infektion in einem Probanden, wobei das Verfahren das Ausführen des Verfahrens von Anspruch 10 umfasst, wobei der nachfolgende Zeitpunkt in Schritt (b) eine Folgetherapie ist, und wobei eine signifikante Differenz der Level der Expression der ps20-Marker in der zweiten Probe in Bezug zu der ersten Probe eine Indikation dafür ist, dass die Therapie wirksam ist, um diese virale Infektion des Probanden zu inhibieren.

13. Verfahren zur Bestimmung des Schweregrades einer viralen Erkrankung in einem Patienten, aufweisend die Schritte des (a) Kontaktierens einer *in vitro* biologischen Probe, die von einem Patienten erhalten wurde, mit einem oder mehreren bindenden Agenzien, die spezifisch an eines oder mehrere ps20-Polypeptide binden, und (b) in der Probe Detektierens einer Menge der ps20-Polypeptide, die an das bindende Agens binden, verglichen mit einem vorgegebenen Standard oder Anhaltewert, und hierauf Bestimmen des Schweregrades einer viralen Erkrankung des Patienten.

14. Verfahren nach irgendeinem der Ansprüche 10 bis 13, wobei die virale Erkrankung HIV oder Influenza ist.

15. Verfahren nach Anspruch 13, wobei die virale Erkrankung HIV ist.

## Revendications

1. Procédé d'aide au diagnostic d'une infection au virus du syndrome immunodéficitaire humain (VIH) chez un sujet, ledit procédé comprenant
(i) la fourniture d'un échantillon *in vitro* provenant du sujet
(ii) la détermination du niveau de ps20 dans ledit échantillon
(iii) la comparaison du niveau de ps20 de (ii) au niveau de ps20 dans un échantillon de référence non infecté,
dans lequel un niveau supérieur de ps20 dans l'échantillon provenant du sujet par comparaison à l'échantillon de référence non infecté indique une probabilité accrue d'une infection au virus du syndrome immunodéficitaire humain chez ledit sujet.

2. Procédé selon la revendication 1, dans lequel l'échantillon comprend un échantillon de lymphocytes T CD4+ provenant du sujet, et dans lequel la détermination du niveau de ps20 dans ledit échantillon comprend la détermination du niveau d'expression de ps20 desdits lymphocytes T.

3. Procédé selon la revendication 1, dans lequel l'échantillon comprend un échantillon de plasma sanguin provenant dudit sujet.

4. Procédé selon la revendication 3, dans lequel le niveau de ps20 est déterminé par un essai ELISA de l'échantillon de plasma sanguin.

5. Procédé selon la revendication 1, dans lequel l'échantillon comprend un échantillon d'acide nucléique provenant dudit sujet, et dans lequel la détermination du niveau de ps20 dans ledit échantillon comprend la détermination de la quantité de transcription de ps20 présente dans ledit échantillon.

6. Procédé d'évaluation de la sensibilité d'un sujet à une infection au virus du syndrome immunodéficitaire humain, ledit procédé comprenant
(i) la fourniture d'un échantillon *in vitro* provenant du sujet
(ii) la détermination du niveau de ps20 dans ledit échantillon
(iii) la comparaison du niveau de ps20 de (ii) au niveau de ps20 dans un échantillon de référence normal,
dans lequel un niveau supérieur de ps20 dans l'échantillon provenant du sujet par comparaison à l'échantillon de référence normal indique une sensibilité accrue à une infection au virus du syndrome immunodéficitaire humain chez ledit sujet.

7. Procédé selon la revendication 6, dans lequel l'échantillon *in vitro* est un échantillon *in vitro* de lymphocytes T CD4+ provenant du sujet, et dans lequel la détermination du niveau de ps20 comprend la détermination du niveau d'expression de ps20 dans lesdits lymphocytes T.

8. Procédé selon l'une quelconque des revendications 1 à 4, de la revendication 6 ou de la revendication 7, dans lequel le niveau d'expression de ps20 est déterminé par l'évaluation d'une liaison par un anticorps anti-ps20.

9. Procédé selon la revendication 9, dans lequel ledit anticorps anti-ps20 comprend un anticorps IG7 monoclonal.

10. Procédé de surveillance de la progression d'une maladie virale chez un sujet, le procédé comprenant : (a) la détection dans un échantillon *in vitro* provenant du sujet à un premier instant dans le temps, d'un ou de plusieurs polypeptides de ps20 ou polynucléotides de ps20 ; (b) la répétition de l'étape (a) à un instant ultérieur dans le temps ; et (c) la comparaison des niveaux détectés lors des étapes (a) et (b), et grâce à cela la surveillance de la progression de la maladie virale.

11. Procédé selon la revendication 10, dans lequel l'étape (a) comprend la mise en contact d'anticorps qui se lient à un ou à plusieurs polypeptides de ps20 avec un échantillon *in vitro* provenant du sujet de manière à former des complexes comprenant les anticorps et un ou plusieurs polypeptides de ps20 dans l'échantillon et la détermination ou la détection de la présence ou d'une quantité d'une formation de complexe dans l'échantillon ; et dans lequel une différence de quantité de formation de complexe est indicative d'une maladie, d'un stade de maladie, et/ou d'une progression de la maladie chez ledit individu.

12. Procédé d'évaluation de l'efficacité d'une thérapie destinée à inhiber une infection virale chez un sujet, le procédé comprenant la réalisation du procédé selon la revendication 10, dans lequel l'instant ultérieur dans le temps de l'étape (b) suit une thérapie et dans lequel une différence significative des niveaux d'expression des marqueurs ps20 dans le deuxième échantillon, par rapport au premier échantillon, est une indication que la thérapie est efficace en ce qui concerne l'inhibition de l'infection virale chez le sujet.

13. Procédé de détermination de la sévérité d'une maladie virale, chez un patient, comprenant les étapes de (a) mise en contact d'un échantillon biologique *in vitro* obtenu à partir d'un patient avec un ou plusieurs agents de liaison qui se lient spécifiquement à un ou à plusieurs polypeptides de ps20 ; et (b) détection dans l'échantillon d'une quantité de polypeptides de ps20 qui se lient à l'agent de liaison, par rapport à une norme prédéterminée ou à une valeur isolée prédéterminée, et à partir de cela détermination de la sévérité d'une maladie virale chez le patient.

14. Procédé selon l'une quelconque des revendications 10 à 13, dans lequel la maladie virale est le VIH ou une grippe.

15. Procédé selon la revendication 13, dans lequel la maladie virale est le VIH.
